# EUROPÄISCHE PATENTSCHRIFT

(11) **EP 4 337 104 B1**
(45) Veröffentlichungstag und Bekanntmachung des Hinweises auf die Patenterteilung: **15.10.2025**
(21) Anmeldenummer: 22724416.7
(22) Anmeldetag: 10.05.2022
(51) Int. Cl.: A61B 10/02, A61B 1/04, A61B 1/06, A61B 5/00, A61B 10/04

(54) **BIOPSIESYSTEM**
BIOPSY SYSTEM
SYSTÈME DE BIOPSIE

(30) Priorität: 14.05.2021 DE 102021204906
(43) Veröffentlichungstag der Anmeldung: 20.03.2024
(73) Patentinhaber: Richard Wolf GmbH, 75438 Knittlingen (DE)
(72) Erfinder: WEBER, Bernd Claus, 76228 Karlsruhe (DE); HÄHNLE, Friedrich, 75015 Bretten (DE); LAMBERTZ, Matthias, 75015 Bretten (DE); HÜTTENBERGER, Dirk, 66887 Rammelsbach (DE)
(74) Vertreter: Patentanwälte Hemmer Lindfeld Frese Partnerschaft mbB
(86) Internationale Anmeldenummer: PCT/DE2022/200095
(87) Internationale Veröffentlichungsnummer: WO 2022/237941

(56) Entgegenhaltungen:
- DE-A1- 102011 122 602
- US-A- 5 983 125
- US-A1- 2016 030 022

## Beschreibung

Die vorliegende Offenbarung betrifft ein Biopsiesystem zur Entnahme von Gewebeproben eines organischen Körpers, insbesondere zur pathologischen Untersuchung.

Häufig wird im Rahmen der Krebsvorsorge oder -nachsorge oder bei einem Krebsverdachtsfall mittels einer Röntgen-, CT- oder UltraschallUntersuchung oder mit Hilfe eines anderen nicht-invasiven bildgebenden Verfahrens eine Abnormität diagnostiziert, die sich beispielsweise als Verschattung im Untersuchungsbild darstellt. Für die Durchführung einer solchen Untersuchung gibt es unterschiedliche Anlässe, z.B. Vorsorge (z.B. Screening der Lunge von Rauchern), Nachsorge (z.B. Untersuchung der Lunge in der Nachsorge eines kolorektalen Karzinoms), oder eine Untersuchung aus einem anderem Anlass (z.B. Untersuchung einer potenziell verletzten Lunge nach einem Unfall mit Zufallsbefund "Rundherd").

Häufig muss der Detektion einer Abnormität eine Behandlung (Therapie) folgen. Voraussetzung für einen gezielten und effizienten therapeutischen Eingriff bzw. die Entscheidung darüber, ob ein solcher therapeutischer Eingriff überhaupt notwendig ist, ist jedoch die Klärung der genauen Ursache der Abnormität im Untersuchungsbild. Die Ursache für die Verschattung kann nämlich völlig unterschiedlicher Art sein, z.B. Tumor, Infektion, Granulom, usw. Unterschiedliche Ursachen erfordern allerdings teilweise völlig unterschiedliche therapeutische Vorgehensweisen (chirurgische Entfernung, Chemotherapie, Strahlentherapie, hormonelle Behandlung, medikamentöse Behandlung, usw.).

Eine solche Klärung (also die Suche nach der Ursache für das abnorme Erscheinungsbild, d.h. für die Verschattung) geschieht über eine ex situ histopathologische Untersuchung des verdächtigen Gewebes. Für die pathologische Untersuchung benötigt man ein Stück Gewebe aus dem Bereich der detektierten Abnormität.

Ein solches Gewebestück wird über eine Gewebeentnahme, eine sogenannte Biopsie, gewonnen. Für die Durchführung der Gewebeentnahme gibt es prinzipiell unterschiedliche Möglichkeiten. Soll der Vorgang wenig patientenbelastend sein, dann kann die Biopsie beispielsweise perkutan durchgeführt werden (z.B. CT- oder ultraschallgesteuert, um den verdächtigen Bereich, wo die Probe entnommen werden soll, sichtbar zu machen). Dabei wird eine dünne, lange, starre Biopsienadel von außen durch die Haut und das darunterliegende Gewebe in das betroffene Organ zu der Abnormität geschoben, um dort, an oder innerhalb der Abnormität, eine Gewebeprobe zu entnehmen. Üblicherweise kommen bei der perkutanen Vorgehensweise Hohlnadeln unterschiedlicher Form und Ausführung zum Einsatz.

Die mit den üblichen bildgebenden Verfahren (CT, Ultraschall, usw.) gewonnenen Bilder ermöglichen zwar das Sichtbarmachen von suspektem Gewebe. Jedoch ist der Grad der Gewebe-Differenzierung eingeschränkt, d.h. die Spezifität ist begrenzt.

Eine Abnormität als solche hebt sich zwar relativ deutlich - beispielsweise durch einen anderen Farbton (bei der CT- und US-Darstellung durch einen anderen Grauton) - vom umgebenden gesunden Gewebe ab. Sie kann dadurch vergleichsweise gut detektiert und die gewünschte Entnahme von Gewebe vergleichsweise einfach durchgeführt werden, indem das distale Ende der Biopsienadel beispielsweise in das Zentrum der Läsion geführt wird.

Jedoch kann mit den üblichen bildgebenden Verfahren (CT, US, usw.) keine Aussage darüber gemacht werden, wie bzw. in welcher Form das verschattet dargestellte Gewebe verändert ist, beispielsweise gutartig oder bösartig. So kann beispielsweise eine bereits in der Vergangenheit behandelte Läsion in CT-Aufnahmen in der Nachsorge weiterhin als Verschattung erscheinen, obwohl es sich dabei nur um fibrös verändertes, also nichtmalignes Gewebe handelt. Eine Biopsie aus dem Zentrum der Verschattung und die nachfolgende pathologische Untersuchung würde diese Diagnose bestätigen.

Ein ggf. immer noch vorhandener Resttumor (aufgrund von beispielsweise unzureichender Erstbehandlung) oder gar eine Tumorneubildung (Rezidiv, neu entstanden nach der Erstbehandlung) könnte im CT- oder US-Bild, vom umgebenden fibrös veränderten und als Verschattung dargestellten Gewebe nicht differenziert werden und bliebe damit unauffällig.

Eine einzelne Biopsie aus dem Zentrum der scheinbar homogen Abnormität - es gäbe anhand des CT- oder US-Bildes zunächst keinen Anlass, an einer anderen Stelle zu biopsieren - würde also zu einer folgenschweren Falschbewertung führen, weil sie den im CT- oder US-Bild nicht differenzierbaren Resttumor oder die Tumorneubildung nicht mit erfassen würde und dementsprechend die tatsächlich erforderliche Therapie ausbleiben würde.

Mit den üblichen bildgebenden Verfahren (CT, US, usw.) kann zudem nicht gewährleistet werden, dass tatsächlich das gesamte suspekte Gewebe sichtbar gemacht wird. Zusätzlich zur Spezifität ist also auch die Sensitivität begrenzt. Das heißt, dass eine Abnormität, die genügend groß ist und deren Gewebe genügend stark verändert ist, sich zwar relativ deutlich als Verschattung vom umgebenden gesunden Gewebe abhebt. Es kann in dem Fall relativ einfach Gewebe aus diesem Bereich entnommen werden und der weiteren pathologischen Untersuchung zugeführt werden. Aber frühmaligne Veränderungen oder kleine maligne Veränderungen lassen sich oft nicht oder zumindest nicht gut genug mit den üblichen bildgebenden Verfahren (CT, US, usw.) darstellen. Ggf. zusätzlich vorhandene frühmaligne Veränderungen und kleine maligne Veränderungen, die beispielsweise im Randbereich der Abnormität oder gar außerhalb der Abnormität liegen, blieben in der CT- oder US-Darstellung, unsichtbar.

Eine Therapie (auf der Basis des histopathologischen Ergebnisses von der Biopsie aus dem zentralen Bereich der Verschattung) würde daher auf das Gebiet der Verschattung und einen "Sicherheitsbereich" um die Verschattung herum begrenzt werden. Etwaige frühmaligne und kleine maligne Veränderungen im Randbereich oder außerhalb der Verschattung (im CT- oder US-Bild unsichtbar) würden nur unzureichend oder gar nicht therapiert werden.

Um dieses Problem zu lösen, ist es bekannt, eine Vielzahl von Biopsien vom gesamten Bereich der Abnormität und auch von dem die Abnormität umgebenden Gewebe zu entnehmen, um das Risiko zu reduzieren, dass pathologisch relevantes Gewebe, das der pathologischen Untersuchung zugeführt werden muss, übersehen wird.

Beispielsweise beschreibt die US 5,938,125 A ein Verfahren und ein Gerät zur Untersuchung von subkutanem Gewebe mit einer optischen Sonde, die durch eine Hohlnadel eingeführt wird, wobei nach der Untersuchung mit der optischen Sonde eine Biopsienadel durch die Hohlnadel eingeführt wird. Die DE 10 2011 122 602 A1 beschreibt eine Vorrichtung und ein Verfahren zur endoskopischen Fluoreszenzdetektion. Die US 2016/0030022 A1 beschreibt ein System und ein Verfahren zur interventionellen optischen molekularen Bildgebung zur Verwendung mit einer Biopsienadel.

Eine Vielzahl von Biopsien hat allerdings viele Nachteile und belastet den Patienten erheblich. Erschwerend, d.h. weiter patienten-belastend kommt hinzu, dass eine gute (im Sinne einer aussagekräftigen) Gewebeprobe ausreichend groß sein sollte und deshalb die Biopsienadel und direkt damit zusammenhängend auch der Einstichkanal einen ausreichend großen Durchmesser erfordern.

Außerdem ist die Entnahme vieler Gewebeproben zeitaufwändig, was neben der Belastung der Bedienperson und der Behandlungskosten eine weitere Belastung für den Patienten bedeutet, z.B. längere Narkotisierung. Hinzu kommt die mit zunehmender Zeit zunehmende Strahlenbelastung bei einer CT-gestützten Gewebeentnahme. Da die Gewebeproben allesamt histopathologisch ausgewertet werden müssen, ist eine solche Vorgehensweise auch kostenintensiv.

Es ist daher eine Aufgabe der vorliegenden Offenbarung, ein Biopsiesystem bereitzustellen, welches es erlaubt, mit weniger Biopsien schneller, kostengünstiger und patientenschonender mit höherer Spezifität und höherer Sensitivität zu einer Diagnose zu kommen.

Gemäß der vorliegenden Offenbarung wird zur Lösung dieser Aufgabe ein Biopsiesystem nach Anspruch 1 bereitgestellt.

Mit einem solchen Biopsiesystem kann das Gewebe bereits vor der tatsächlichen Entnahme für die pathologische Untersuchung in situ vorselektiert werden. Das bedeutet, dass das mit den bekannten nicht-invasiven bildgebenden Verfahren (CT, US, usw.) undifferenziert suspekt erscheinende Gewebe bereits in situ differenzierter (z.B. hinsichtlich der Malignität) bewertet werden kann, wodurch die Spezifität erhöht wird. Durch das Fluoreszenz-Endoskopieelement kann Gewebe mit Anregungslicht in situ zur Fluoreszenz angeregt werden. Dabei kann das mittels Anregungslicht angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise mittels eines Photosensibilisators bzw. Markerstoffs (z.B. Chlorin e6) durchgeführt werden, der sich selektiv an pathologischem Gewebe anreichert und fluoresziert. Es ist aber auch möglich, auf einen solchen Photosensibilisator zu verzichten und stattdessen die Fluoreszenz von gewebeeigenen Farbstoffen zu detektieren (sogenannte Autofluoreszenz), wobei sich diese Fluoreszenz abhängig vom Gewebezustand unterschiedlich darstellt.

Mit dem Fluoreszenz-Endoskopieelement kann also sehr genau tatsächlich pathologisches Gewebe lokalisiert werden. Wenn man mittels des Fluoreszenz-Endoskopieelements anhand der Fluoreszenz für die Biopsie relevantes Gewebe lokalisiert hat, kann das Fluoreszenz-Endoskopieelement aus der Einführhülse herausgezogen werden, wobei die Einführhülse im Körper verbleibt. Die Biopsienadel kann dann durch die Einführhülse genau an die Stelle geführt werden, wo das Fluoreszenz-Endoskopieelement das zu untersuchende Gewebe erkannt hat. Es können also mit diesem Biopsiesystem in situ mehr, idealerweise alle relevanten, z.B. frühmalignen und malignen, Bereiche im betreffenden Organ lokalisiert werden, ohne dafür patientenbelastend willkürliche Zufallsbiopsien von nicht relevantem Gewebe entnehmen zu müssen. Das bedeutet eine erheblich höhere Sensitivität.

Die Anzahl der Gewebeproben, die dann am Ende tatsächlich entnommen und ex situ pathologisch untersucht werden müssen, werden mit diesem Biopsiesystem stark reduziert, was die Diagnose deutlich schneller, patientenschonender und kostengünstiger macht.

Ein weiterer Vorteil dieses Biopsiesystems ist es, dass damit das Risiko verringert wird, dass die entnommene Gewebeprobe, die pathologisch als relevantes Gewebestück untersucht wird und über die im Anschluss erfolgende therapeutische Vorgehensweise entscheidet, tatsächlich auch exakt an der Stelle entnommen wird, welche in situ als relevant (als mit hoher Wahrscheinlichkeit tatsächlich frühmaligne/maligne) vorselektiert wurde. Mit anderen Worten ist die Gefahr bei diesem Biopsiesystem geringer, dass man mit dem Entnahme-Instrumentarium (Stanze, Zange, Biopsienadel usw.) daneben greift.

Optional kann die Einführhülse biegesteifer sein als der distale Abschnitt des Fluoreszenz-Endoskopieelements. Vorzugsweise kann der distale Abschnitt des Fluoreszenz-Endoskopieelements erheblich dünner ausgestaltet sein als das Biopsieelement. Es kann vorteilhaft sein, wenn die Einführhülse zusammen mit dem Fluoreszenz-Endoskopieelement beispielsweise perkutan in den Körper eingestochen und in Richtung des zu untersuchenden Gewebes geführt wird. Die Einführhülse kann dann den distalen Abschnitt des Fluoreszenz-Endoskopieelements stabilisieren, insbesondere beim Durchstechen von festem Gewebe, z.B. Haut und Muskein. Die Einführhülse kann, aber muss axial nicht bis in das zu untersuchende Gewebe reichen. Der distale Abschnitt des Fluoreszenz-Endoskopieelements kann durch weicheres Gewebe über das distale Ende der Einführhülse hinaus in das zu untersuchende Gewebe gestochen werden. Der distale Abschnitt des Fluoreszenz-Endoskopieelements sollte dabei zumindest so biegesteif sein, dass er sich beim Durchstechen von weicherem Gewebe nicht oder nur wenig verbiegt. In der Länge kann die Einführhülse dem zu untersuchenden Organ angepasst sein oder eine organspezifische Einführhülse mit einer bestimmten Länge für die Untersuchung eines entsprechenden Organs ausgewählt werden.

Alternativ dazu kann das Fluoreszenz-Endoskopieelement selbst relativ biegesteif sein, beispielsweise kann der distale Abschnitt des Fluoreszenz-Endoskopieelements in einem dünnwandigen Rohr geführt sein, das den distalen Abschnitt des Fluoreszenz-Endoskopieelements verstärkt. Der Einstechvorgang des distalen Abschnitts des Fluoreszenz-Endoskopieelements kann dann zunächst ohne Hilfe der Einführhülse erfolgen. Wenn allerdings die finale Entnahmeposition mittels des Fluoreszenz-Endoskopieelements gefunden wurde, kann die Einführhülse von proximal über den distalen Abschnitt des Fluoreszenz-Endoskopieelements geschoben und distalwärts bei in situ positioniertem distalen Abschnitt des Fluoreszenz-Endoskopieelements eingestochen werden. Sobald die Einführhülse in situ final positioniert ist, wird der distale Abschnitt des Fluoreszenz-Endoskopieelements proximalwärts herausgezogen, wobei die Einführhülse in situ final positioniert bleibt. Sobald der distale Abschnitt des Fluoreszenz-Endoskopieelements vollständig aus der Einführhülse herausgezogen ist, wird das Biopsieelement zur Gewebeentnahme durch die Einführhülse in oder an das zu untersuchende Gewebe geführt.

Optional kann die Einführhülse proximalseitig distalwärts über den distalen Abschnitt des Fluoreszenz-Endoskopieelements stülpbar sein. Dies ist insbesondere dann vorteilhaft, wenn das Fluoreszenz-Endoskopieelement selbst relativ biegesteif ist und die Einführhülse nicht dazu benötigt, um den distalen Abschnitt des Fluoreszenz-Endoskopieelements beim Einstich in das Gewebe zu stabilisieren. Dann kann nämlich der Einsatz der Einführhülse so lange zurückgehalten werden, bis die endgültige Positionierung des Fluoreszenz-Endoskopieelements gefunden ist. Dann kann beispielsweise eine proximalseitig am distalen Abschnitt des Fluoreszenz-Endoskopieelements angeschlossene optische Komponenten abgekoppelt werden, um die Einführhülse, die in diesem Fall sogar biegsamer als der verstärkte distale Abschnitt des Fluoreszenz-Endoskopieelements sein kann, proximalseitig distalwärts über den distalen Abschnitt des Fluoreszenz-Endoskopieelements gestülpt bzw. geschoben werden. Die Einführhülse wird dann bei bereits eingestochenem distalen Abschnitt des Fluoreszenz-Endoskopieelements zusätzlich in das Gewebe eingestochen, wodurch sich die Gewebeöffnung aufweitet. Dies ist eine besonders gewebeschonende Vorgehensweise, um später durch die Einführhülse, bei herausgezogenem distalen Abschnitt des Fluoreszenz-Endoskopieelements eine dickere Biopsienadel in das Gewebe einzuführen.

Erfindungsgemäß hat das Biopsieelement eine größere radiale Ausdehnung als der distale Abschnitt des Fluoreszenz-Endoskopieelements und ein Innendurchmesser der Einführhülse zur wahlweisen Aufnahme des distalen Abschnitts des Fluoreszenz-Endoskopieelements und des Biopsieelements ist anpassbar. Dies ist besonders vorteilhaft, da die Vorselektion des Gewebes mittels des Fluoreszenz-Endoskopieelements weniger invasiv ist als das Einstechen des Biopsieelements. Bei der Vorselektion kann es vorkommen, dass mit dem Fluoreszenz-Endoskopieelement kein malignes Gewebe erkannt wird und an anderer Stelle weitergesucht werden muss. In diesem Fall hat man sich die vergleichsweise patientenbelastende Gewebeentnahme mit dem Biopsieelement an nicht relevanter Stelle gespart. Der Einstich mit dem dünneren distalen Abschnitt des Fluoreszenz-Endoskopieelements an nicht relevanter Stelle ist dagegen patientenschonender.

Optional kann die Einführhülse axial geschlitzt und aufweitbar sein, vorzugsweise über die gesamte Länge der Einführhülse. Die Einführhülse ist vorzugsweise weitestgehend elastisch aufweitbar und beispielsweise aus Metall. Ein Aufspreizen der Einführhülse beim Einführen des Biopsieelements ist patientenschonender als ein direkter Einstich mit dem Biopsieelement.

Optional kann die Einführhülse zur Aufnahme des distalen Abschnitts des Fluoreszenz-Endoskopieelements in Umfangsrichtung überlappend ausgeführt sein, wobei die Überlappung bei aufgenommenem Biopsieelement geringer ist oder nicht mehr besteht. Damit kann die Gefahr von Gewebeeinklemmungen reduziert werden.

Optional kann die Einführhülse eine Innenhülse und eine Außenhülse aufweisen, wobei zur Aufnahme des Biopsieelements die Innenhülse aus der Außenhülse proximalwärts entfernbar ist. Die Einführhülse kann also zur Aufnahme des distalen Abschnitts des Fluoreszenz-Endoskopieelements zumindest zwei Teile aufweisen, nämlich zumindest eine Innenhülse und eine Außenhülse, die ineinander steckbar, lösbar voneinander und gegeneinander verschiebbar sind.

Optional weist dabei die Innenhülse einen Innendurchmesser auf, der im Wesentlichen einem Außendurchmesser des distalen Abschnitts des Fluoreszenz-Endoskopieelements entspricht, und die Außenhülse einen Innendurchmesser aufweist, der im Wesentlichen einem Außendurchmesser des Biopsieelements entspricht. Genau genommen ist dabei der Innendurchmesser der Innenhülse geringfügig größer als der Außendurchmesser des distalen Abschnitts des Fluoreszenz-Endoskopieelements und der Innendurchmesser der Außenhülse geringfügig größer als der Außendurchmesser des Biopsieelements. Die Innenhülse kann ggf. länger als die Außenhülse sein. Die Außenhülse muss nur so tief eingestochen sein, dass ihre Position im Gewebe stabil ist. Wenn nun eine auffällige Fluoreszenz detektiert wird, kann - unter Beibehaltung der Position der Außenhülse - der distale Abschnitt des Fluoreszenz-Endoskopieelements zusammen mit der Innenhülse aus der Außenhülse gezogen werden und das Biopsieelement in die Außenhülse eingeführt und an den Ort der auffälligen Fluoreszenz vorgeschoben werden. Eine radiale Aufweitung der Einführhülse ist also in der zweiteiligen Ausführungsform der Einführhülse nicht erforderlich

Optional kann sowohl das Fluoreszenz-Endoskopieelement als auch das Biopsieelement Längenmarkierungen und/oder einen axial positionierbaren Anschlag für eine definiert reproduzierbare relative Axialposition des distalen Abschnitts des Fluoreszenz-Endoskopieelements bzw. des Biopsieelements zur Einführhülse aufweisen. Beispielsweise können das Fluoreszenz-Endoskopieelement als auch das Biopsieelement jeweils eine Längenskala aufweisen, welche auf dem Fluoreszenz-Endoskopieelement den Abstand zur distalen Spitze des distalen Abschnitts des Fluoreszenz-Endoskopieelements und beim Biopsieelement den Abstand zur distalen Spitze des Biopsieelements anzeigt. Damit lässt sich dann auf einfache Weise mit dem Biopsieelement genau die axiale Position relativ zur Einführhülse (d.h. in Längsrichtung der Einführhülse) reproduzieren, die der distale Abschnitt des Fluoreszenz-Endoskopieelements zuvor hatte. Vorzugsweise umfassen die Längenmarkierungen Markierungen von besonderen axialen Positionen. Beispielsweise kann die Länge der Einführhülse besonders angezeigt sein, sodass eine Bedienperson erkennt, ob und wie viel das distale Ende des distalen Abschnitts des Fluoreszenz-Endoskopieelements und/oder des Biopsieelement über das distale Ende der Einführhülse hinausragt.

Optional weist die Einführhülse an einem proximalen Ende der Einführhülse einen Flansch auf. Der Flansch kann die Handhabung der Einführhülse und ein vollständiges Eindringen der Einführhülse in die Haut verhindern. Der Anschlag kann beim Einstechen der Einführhülse an die Haut anschlagen und somit die maximale Einstichtiefe der Einführhülse bestimmen. Vorzugsweise wird die Länge der Einführhülse für ein zu untersuchendes Organ speziell so gewählt, dass bei maximaler Einstichtiefe der Einführhülse, wobei der proximale Anschlag der Einführhülse an die Haut anschlägt, das distale Ende der Einführhülse nicht bis in das zu untersuchende Gewebe eindringt, sondern proximal davor endet. Der eigentliche Einstich in das zu untersuchende Gewebe erfolgt dann nur mit dem über das distale Ende der Einführhülse hinausragenden distalen Ende des distalen Abschnitts des Fluoreszenz-Endoskopieelements bzw. mit dem über das distale Ende der Einführhülse hinausragenden distalen Ende distalen Ende des Biopsieelements.

Optional kann das Fluoreszenz-Endoskopieelement dazu eingerichtet sein, wahlweise ein erstes Anregungslicht und ein zweites Anregungslicht proximal einzukoppeln, wobei das erste Anregungslicht im Mittel kurzwelliger ist als das zweite Anregungslicht, und wobei das Fluoreszenz-Endoskopieelement dazu eingerichtet ist, wahlweise ein erstes Bild oder ein erstes Fluoreszenzsignal von einem ersten Gewebebereich aus mit dem ersten Anregungslicht angeregtem ersten Fluoreszenzlicht zu erzeugen und ein zweites Bild oder ein zweites Fluoreszenzsignal von einem zweiten Gewebebereich aus mit dem zweiten Anregungslicht angeregtem zweiten Fluoreszenzlicht aufzunehmen. Da die Eindringtiefe von dem kurzwelligen ersten Anregungslicht geringer sein kann als die Eindringtiefe von dem langwelligeren zweiten Anregungslicht, ist der erste Gewebebereich vorzugsweise kleiner als der zweite Gewebebereich. Die Lokalisierung von pathologischem Gewebe erfolgt so mit einer höheren räumlichen Auflösung mittels des ersten Anregungslichts. Da die Eindringtiefe von dem langwelligeren zweiten Anregungslicht größer sein kann, kann auch das detektierte Fluoreszenzsignal aus einem entsprechend größeren zweiten Gewebebereich stammen. Die Lokalisierung von pathologischem Gewebe mittels des zweiten Anregungslichts erfolgt so mit einer geringeren räumlichen Auflösung, es wird aber ein größeres Volumen erfasst. Es kann also zunächst ein größerer zweiter Gewebebereich grobmaschiger mit dem langwelligeren zweiten Anregungslicht vorselektiert werden und dann ein kleinerer erster Gewebebereich, der vorzugsweise ein Teil des zweiten Gewebebereichs ist, engmaschiger mit dem kurzwelligen ersten Anregungslicht untersucht werden.

Optional kann das Fluoreszenz-Endoskopieelement eine proximalseitig mit dem distalen Abschnitt des Fluoreszenz-Endoskopieelements lösbar koppelbare oder fest gekoppelte optische Komponente aufweisen, wobei die optische Komponente einen Strahlteiler mit einer Bildpfadseite und einer Beleuchtungspfadseite aufweist, wobei zum Auskoppeln des Fluoreszenzlichts ein Bildsensor, eine Photodiode o.ä. mit der Bildpfadseite lösbar koppelbar oder fest gekoppelt ist, und wobei zum Einkoppeln des Anregungslichts eine Lichtquelle mit der Beleuchtungspfadseite lösbar koppelbar oder fest gekoppelt ist. Die Lichtquelle ist vorzugsweise mindestens eine LED oder mindestens eine Laserauskopplung. Der Strahlteiler kann beispielsweise ein Prismenpaar aufweisen, das zusammen einen Strahlteilerblock bildet und das Anregungslicht und das Fluoreszenzlicht zumindest zu einem Großteil voneinander trennt, sodass das Fluoreszenzlicht zur Bildpfadseite transmittiert oder reflektiert wird. Entsprechend anders herum kann das Prismenpaar das Anregungslicht zur Beleuchtungspfadseite reflektieren bzw. transmittieren. Vorzugsweise liegen die Bildpfadseite und die Beleuchtungspfadseite des Strahlteilers an in einem Winkel, vorzugsweise von etwa 90°, zueinander angeordneten Seiten des Strahlteilers. Vorzugsweise liegt die Bildpfadseite proximalseitig, sodass Fluoreszenzlicht von der distalen Eintrittsseite des Strahlteilers zur Bildpfadseite im Wesentlichen transmittiert wird. Die Beleuchtungspfadseite liegt vorzugsweise lateralseitig, sodass Anregungslicht von der lateralen Eintrittsseite zur distalen Austrittseite hin im Wesentlichen reflektiert wird.

Es sei an dieser Stelle angemerkt, dass "Bildpfad" hier nicht darauf beschränkt ist, dass aus dem eingekoppelten Fluoreszenzlicht ein Bild erzeugt wird. Es kann im Bildpfad lediglich ein eingekoppeltes Fluoreszenzsignal übertragen werden, dessen Eigenschaft bestimmt wird. Eine Einkopplung von Fluoreszenzlicht mittels eines Bildsensors, einer Photodiode o.ä. muss also hier nicht unbedingt zur Bilderzeugung verwendet werden. Für eine Entscheidung, ob das Fluoreszenzsignal auffällig ist oder nicht, d.h. eine Biopsie entnommen werden soll oder nicht, ist es oft hinreichend, wenn lediglich eine Eigenschaft eines Fluoreszenzsignals, beispielsweise dessen Amplitude oder Intensität, bestimmt wird. Beispielsweise kann sich die Auffälligkeit in einer gegenüber dem umliegenden Gewebe oder gegenüber einem Referenzwert erhöhten oder niedrigeren Fluoreszenz-Intensität zeigen.

Optional kann die optische Komponente ein optisches Langpassfilter aufweisen, welches das Fluoreszenzlicht stärker zur Bildpfadseite transmittiert als das Anregungslicht, und/oder ein optisches Kurzpassfilter aufweist, welches das Fluoreszenzlicht stärker zur Bildpfadseite reflektiert als das Anregungslicht. Beispielsweise kann das Langpassfilter bzw. das Kurzpassfilter zwischen einem als Strahlteiler fungierenden Prismenpaar angeordnet sein und/oder als Schicht auf einer oder beiden der zueinander gewandten Seiten der Prismen realisiert sein. Dies ist insbesondere dann sinnvoll, wenn das Anregungslicht kurzwelliger ist als das Fluoreszenzlicht.

Optional kann das Fluoreszenz-Endoskopieelement eine mit dem Bildsensor, der Photodiode o.ä. und/oder der Lichtquelle signalverbundene oder -verbindbare Betriebseinheit aufweisen, wobei die Betriebseinheit eine optische und/oder akustische Anzeigekomponente, eine Steuer- und Versorgungskomponente und eine Bedienungskomponente aufweist.

Optional kann das Fluoreszenz-Endoskopieelement bzw. die optische Komponente mit einem Handgriff oder einer vergleichbaren ergonomischen Komponente versehen sein, sodass sie zusätzlich auch als Handhabungseinrichtung zum Einführen des distalen Abschnitts des Fluoreszenz-Endoskopieelements in die Haut und das darunterliegende Gewebe verwendet werden kann und deshalb auf eine solche Handhabungseinrichtung als separate und zusätzliche Einheit, die nach dem Einführen des Lichtleiters in das Gewebe vom distalen Abschnitt des Fluoreszenz-Endoskopieelements wieder entfernt werden muss, um die optische Komponente ankoppeln zu können, verzichtet werden kann.

Optional kann das Biosiesystem ferner eine bezüglich des organischen Körpers positionierbare und fixierbare Schablone mit mindestens einer Durchbrechung zur selektiven Aufnahme der Einführhülse und des Biopsieelements aufweisen, wobei die Einführhülse und/oder das Biopsieelement in der mindestens einen Durchbrechung gegen laterale Verschiebung und Verkippung durch die Schablone fixiert ist. Dies ist besonders sinnvoll, wenn die Haut des Patienten an der zu durchstechenden Köperstelle nicht fest genug ist, um die Einführhülse sicher bezüglich des zu untersuchenden Gewebes zu fixieren. Die Schablone kann alternativ oder zusätzlich zur Haut zur Fixierung der Einführhülse bzw. des Biopsieelements genutzt werden.

Eine solche Schablone kann eine flächige oder dreidimensionale Struktur sein, die starr ist oder zumindest wesentlich steifer als die Haut eines Patienten. Die Schablone kann beispielsweise auf die Haut des Patienten aufgeklebt oder aufgeschnallt werden und somit relativ zu dem zu untersuchenden Gewebe fixiert werden. Alternativ oder zusätzlich kann die Schablone bezüglich eines Referenzpunkts, beispielsweise auf einem Operationstisch, fixiert sein. Der Patient kann dann ebenfalls bezüglich dieses Referenzpunkts, beispielsweise auf dem Operationstisch, fixiert sein. Die Schablone kann speziell auf ein zu untersuchendes Organ passend konzipiert sein, sodass die mindestens eine Durchbrechung der Bedienperson nur noch einen Bewegungsfreiheitsgrad für den distalen Abschnitt des Fluoreszenz-Endoskopieelements bzw. das Biopsieelement erlaubt, nämlich die Einstichtiefe. Die laterale Positionierung und Ausrichtung sind durch die mindestens eine Durchbrechung der Schablone festgelegt. Die Einführhülse muss dann gar nicht mehr in den Körper des Patienten gestochen werden, sondern nur noch in die Durchbrechung, wo sie lediglich als eine Art Adapter dient, um die Dickenunterschiede zwischen dem distalen Abschnitt des Fluoreszenz-Endoskopieelements und dem Biopsieelement auszugleichen.

Optional kann die Einführhülse dabei einen ersten Teil und einen zweiten Teil aufweisen, wobei der erste Teil den distalen Abschnitt des Fluoreszenz-Endoskopieelements in der mindestens einen Durchbrechung gegen laterale Verschiebung und Verkippung fixiert, und wobei der zweite Teil das Biopsieelement in der mindestens einen Durchbrechung gegen laterale Verschiebung und Verkippung fixiert. Die beiden Teile können in Form von ineinandergesteckter Innen- und Außenhülse ausgeführt sein oder separat jeweils den distalen Abschnitt des Fluoreszenz-Endoskopieelements bzw. das Biopsieelement fixieren. Bei einteiliger Ausgestaltung der Einführhülse kann die Durchbrechung selbst die Funktion des zweiten Teils übernehmen und eine entsprechende Führungsfläche aufweisen, sodass nur der erste Teil der Einführhülse benötigt wird.

Optional kann die Schablone eine Vielzahl von Durchbrechungen aufweisen, welche wahlweise zur Aufnahme der Einführhülse und des Biopsieelements zur Verfügung stehen. Die Schablone weist vorzugsweise ein Raster von Durchbrechungen auf, sodass eine Bedienperson über die Wahl der Durchbrechung und die jeweils gewählte Einstichtiefe malignes Gewebe mit dem wenig traumatischen Fluoreszenz-Endoskopieelement suchen kann, bevor das traumatischere Biopsieelement zum Einsatz kommt. Es können dabei auch mehrere Fluoreszenz-Endoskopieelemente gleichzeitig eingesetzt werden. Sobald durch ein auffälliges Fluoreszenzsignal eine Stelle für die Biopsie gefunden wurde, muss die Bedienperson lediglich die Einstichtiefe anhand von Längenmarkierungen mit dem Biopsieelement reproduzieren. Die Durchbrechungen können im Wesentlichen parallel zueinander oder gewinkelt zueinander ausgerichtet sein. Bei großen Organen, die ggf. größer als die Schablone sind, kann eine parallele Ausrichtung sinnvoll sein. Insbesondere bei Organen, die kleiner als die Schablone sind, beispielsweise der Prostata, können die Durchbrechungen so gewinkelt zueinander ausgerichtet sein, dass diese aus verschiedenen Richtungen auf das Organ weisen. Die Schablone ist also vorzugsweise genau auf das Organ oder ggf. sogar individuell an einzelne Patienten oder bestimmte Patientengruppen angepasst.

Vorzugsweise ist ein Rastermaß, d.h. der Abstand der Durchbrechungen in der Schablone, an die Eindringtiefe des Fluoreszenzanregungslichtes angepasst. Das bedeutet, dass die Durchbrechungen in der Schablone vorzugsweise einerseits höchstens so weit voneinander entfernt sind, dass jedes Volumenelement des zu untersuchenden Organs im Hinblick auf die Detektion von Fluoreszenzsignalen erreicht und erfasst werden kann. Dadurch weiß eine Bedienperson, dass sie das gesamte Organ lückenlos fluoreszenztechnisch erfassen und voruntersuchen kann, wenn sie einfach nur alle Durchbrechungen nutzt. Das bedeutet aber auch, dass die Durchbrechungen in der Schablone andererseits nicht beliebig eng zueinander angebracht werden, um sowohl die Patientenbelastung als auch den Zeitaufwand für die Untersuchung durch eine Reduzierung der Anzahl der Einstichkanäle zu minimieren.

Die Begriffe "distalwärtig" bzw. "proximalwärtig" sollen hierin eine relative Position bedeuten, die sich distal bzw. proximal von einer Bedienperson des Systems als Bezugsposition befindet. Die Begriffe "distalseitig" bzw. "proximalseitig" sollen hierin entsprechend Positionen an einer distalen bzw. proximalen Seite eines Gegenstands bedeuten. Die Begriffe "distalwärts" bzw. "proximalwärts" sollen hierin entsprechend Richtungen bedeuten, die sich nach distal bzw. proximal erstrecken.

Die Offenbarung ist nachfolgend anhand von in den Zeichnungen dargestellten Ausführungsbeispielen näher erläutert. Es zeigen:
Fig. 1a-c drei schematische Längsschnittdarstellungen einer ersten beispielhaften Ausführungsform eines hierin offenbarten Biopsiesystems in jeweils verschiedenen Phasen der Verwendung;
Fig. 2a-c drei weitere schematische Längsschnittdarstellungen der ersten beispielhaften Ausführungsform eines hierin offenbarten Biopsiesystems in weiteren Phasen der Verwendung;
Fig. 3a-c drei schematische Längsschnittdarstellungen einer zweiten beispielhaften Ausführungsform eines hierin offenbarten Biopsiesystems in jeweils verschiedenen Phasen der Verwendung;
Fig. 4a-c drei weitere schematische Längsschnittdarstellungen der zweiten beispielhaften Ausführungsform eines hierin offenbarten Biopsiesystems in weiteren Phasen der Verwendung;
Fig. 5a-c jeweils schematische Querschnittdarstellungen der ersten zweiten und dritten beispielhaften Ausführungsform eines hierin offenbarten Biopsiesystems in jeweils verschiedenen Phasen der Verwendung;
Fig. 6a-c, 7a,b, 8a-c und 9 schematische Längsschnittdarstellungen einer dritten beispielhaften Ausführungsform eines hierin offenbarten Biopsiesystems in jeweils verschiedenen Phasen der Verwendung;
Fig. 10 eine schematische Längsschnittdarstellungen einer Problemstellung der ersten, zweiten und dritten beispielhaften Ausführungsform des hierin offenbarten Biopsiesystems;
Fig. 11 eine schematische Darstellung einer vierten beispielhaften Ausführungsform eines hierin offenbarten Biopsiesystems zur Lösung der in Fig. 10 gezeigten Problemstellung; und
Fig. 12a-c, 13a-c, 14a,b und 15 a,b schematische Längsschnittdarstellungen der vierten beispielhaften Ausführungsform eines hierin offenbarten Biopsiesystems in jeweils verschiedenen Phasen der Verwendung; und
Fig. 16 eine schematische Darstellung ein Fluoreszenz-Endoskopieelement gemäß einem Beispiel eines hierin offenbarten Biopsiesystems,

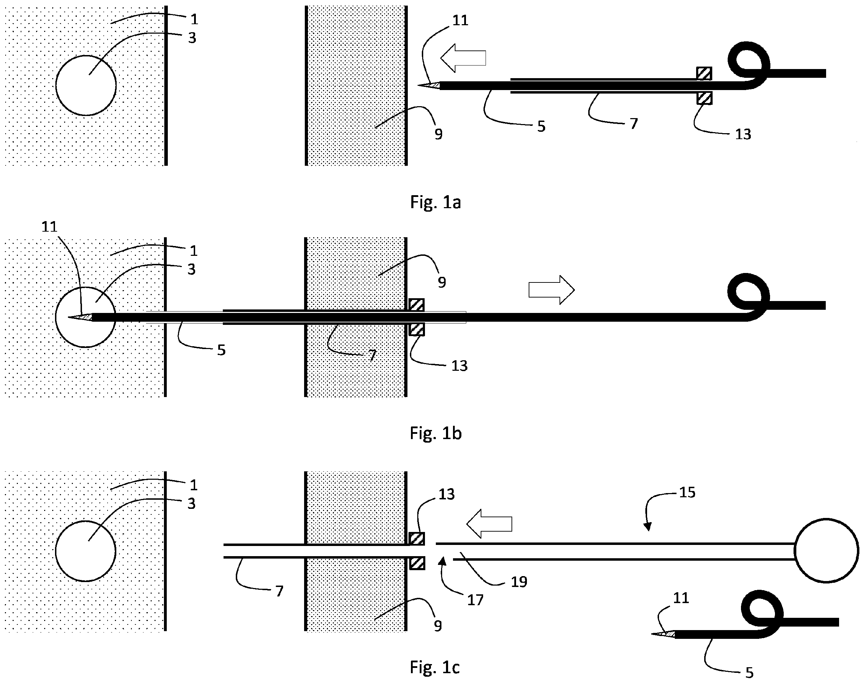

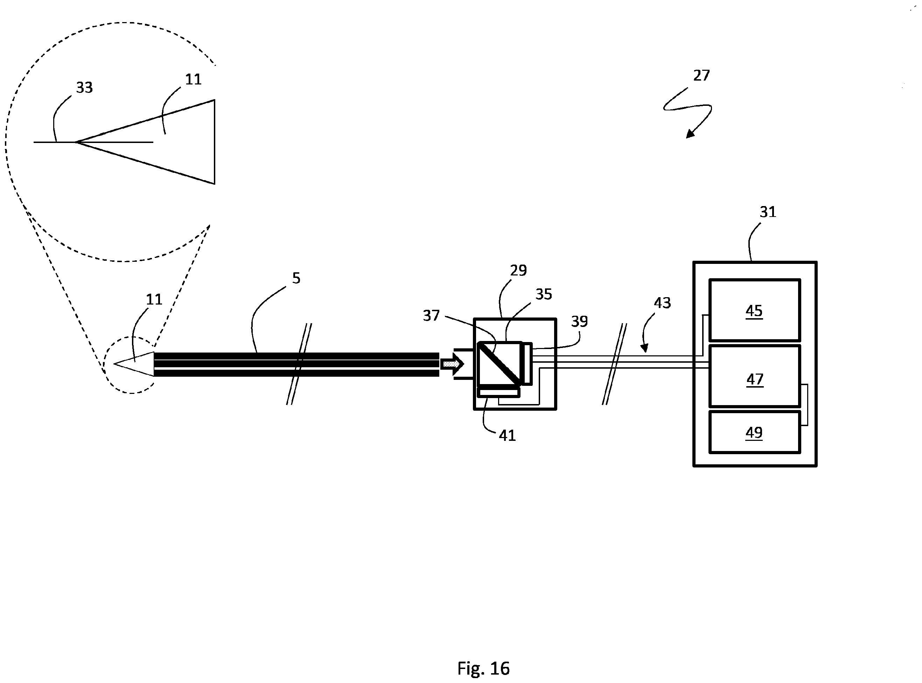

Figuren 1a-c zeigen ein Biopsiesystem in den ersten drei Stufen einer Anwendung. In einem Organ 1 im Körper eines Patienten befindet sich zu untersuchendes Gewebe 3, das beispielsweise in einem Röntgen-CT- und/oder US-Bild auffällig erscheint, beispielsweise als Schatten. Für die genauere Diagnose soll nun eine Gewebeprobe von dem zu untersuchenden Gewebe 3 entnommen werden, um diese ex situ pathologisch untersuchen zu können. Das hier für die Gewebeprobenentnahme (Biopsie) verwendete Biopsiesystem weist einen distalen Abschnitt 5 eines in Fig. 16 gezeigten Fluoreszenz-Endoskopieelements 27 auf, welcher mittels einer Einführhülse 7 perkutan durch die Haut 9 des Patienten in Richtung des zu untersuchendes Gewebes 3 eingestochen werden kann. Wie in Fig. 1a kurz vor dem Einstich gezeigt, ist der distale Abschnitt 5 durch die Einführhülse 7 führbar und relativ zur Einführhülse 7 axial positionierbar. Die axiale Position des distalen Abschnitts 5 kann gewünscht eingestellt und fixiert werden, beispielsweise durch manuellen lateralen Andruck oder eine anderweitige Verklemmung (nicht gezeigt). Der distale Abschnitt 5 weist eine distale Spitze 11 auf, welche beim Einstich über das distale Ende der Einführhülse 7 hinausragt, um den Einstich so atraumatisch wie möglich zu gestalten. Die Einführhülse 7 kann den distalen Abschnitt 5 gegen Verbiegung stabilisieren, insbesondere beim Weg durch gesundes, festes Haut- und/oder Muskelgewebe 9. Auch die Handhabung des distalen Abschnitts 5 wird durch die um den distalen Abschnitt 5 befindliche Einführhülse 7 erleichtert. Der Einstich erfolgt vorzugsweise begleitend mit einer CT- oder US-Aufnahme, sodass beobachtet werden kann, ob der Einstich richtig positioniert und ausgerichtet ist.

In Figur 1b sind der distale Abschnitt 5 und die Einführhülse 7 bereits final positioniert. Ein proximaler Anschlag 13 an der Einführhülse 7 liegt an der Haut 9 außen an, sodass die Einführhülse 7 eine Position maximaler Eindringtiefe erreicht hat. Die Länge der Einführhülse 7 ist dabei so gewählt, dass sie nicht in das Organ 3 selbst reicht, sondern proximal davon endet. Die Einführhülse 7 unterstützt den distalen Abschnitt 5 also vornehmlich auf dem Weg durch festes Hautgewebe 9. Der distale Abschnitt 5 ist hingegen weiter distalwärts in der Einführhülse 7 geschoben worden, sodass die distale Spitze 11 in das Organ 1 und das zu untersuchende Gewebe 3 eindringt. Wie in Fig. 16 genauer erläutert, ist der distale Abschnitt 5 Teil des Fluoreszenz-Endoskopieelements 27 des Biopsiesystems und kann einen Lichtleiter oder ein Lichtleiterbündel zur Anregungslichtauskopplung und/oder Fluoreszenzsignaleinkopplung aufweisen und das Signal optisch übertragen. Der distale Abschnitt 5 weist dabei eine proximalseitige Einkopplung von Anregungslicht in den Lichtleiter und, an der distalen Spitze 11 eine distalseitige Auskopplung des Anregungslichts aus dem Lichtleiter auf. Außerdem weist der distale Abschnitt 5 an der distalen Spitze 11 eine distalseitige Einkopplung von Fluoreszenzlicht in den Lichtleiter und eine proximalseitige Auskopplung des Fluoreszenzlichts aus dem Lichtleiter auf. Das bedeutet, dass während des Einstichs und der axialen Positionierung des distalen Abschnitts 5 relativ zur Einführhülse 7 Anregungslicht an der distalen Spitze 11 distalwärts ausgekoppelt werden kann und gleichzeitig über die distale Spitze 11 Fluoreszenzlicht proximalwärts eingefangen werden kann. Das Anregungslicht kann dabei kurzwelliger sein als das Fluoreszenzlicht. Alternativ oder zusätzlich zu einem Lichtleiter oder Lichtleiterbündel kann der distale Abschnitt 5 des Fluoreszenz-Endoskopieelements 27 distalseitig eine LED zur Anregungslichtauskopplung und ebenfalls distalseitig einen Bildsensor oder eine Photodiode zur Fluoreszenzsignaleinkopplung aufweisen und das Fluoreszenzsignal elektronisch übertragen.

Damit sich malignes Gewebe 3 von gesundem Gewebe durch seine Fluoreszenz deutlich abhebt, kann dem Patienten vor der Biopsie ein Photosensibilisator bzw. Markerstoffs zugeführt werden. Es kann auf das Zuführen eines Photosensibilisators bzw. Markerstoffs ggf. verzichtet werden. Dann wird die Fluoreszenz körpereigener Farbstoffe detektiert, die - abhängig vom Gewebezustand - in unterschiedlichen Konzentrationen vorhanden sind (Autofluoreszenz). Die Höhe der detektierten Fluoreszenz ist auch hier ein Maß für den Gewebezustand. Die Auffälligkeit des Fluoreszenzsignals kann sich beispielsweise in einem gegenüber gesundem Gewebe verstärkten oder abgeschwächten Fluoreszenzsignal zeigen. Es kann also in situ anhand der Amplitude der eingefangenen Fluoreszenz erkannt werden, ob sich die distale Spitze 11 in malignen Gewebe 3 befindet oder nicht. Es kann auch sein, dass das im CT- oder US-Bild als Schatten auffällige Gewebe 3 tatsächlich keine Auffälligkeit in der Fluoreszenz zeigt. In diesem Fall wäre eine Biopsie von dem Gewebe 3 entbehrlich.

Wird jedoch ein auffälliges Fluoreszenzsignal erkannt, so kann durch eine axiale Bewegung der distalen Spitze 11 die Größe bzw. die axiale Mitte des fluoreszierenden Gewebes 3 ermittelt werden, indem die distale Spitze 11 so positioniert wird, dass das empfangene Fluoreszenzsignal maximal auffällig ist. Sodann kann eine Bedienperson die axiale Position des distalen Abschnitts 5 relativ zur Einführhülse 7 beispielsweise an einer Längenmarkierung am distalen Abschnitt 5 ablesen oder markieren. Dann kann der distale Abschnitt 5 proximalwärts aus der Einführhülse 7 gezogen werden, wobei die Einführhülse 7 an Ort und Stelle verbleibt.

In Fig. 1c wird nun für die eigentliche Biopsie ein Biopsieelement 15, beispielsweise in Form einer Biopsie-Hohlnadel, an die Einführhülse 7 angesetzt. Das Biopsieelement 15 weist hier ein abgeschrägtes distales Ende 17 auf, das eine distale Hohlkammer 19 zur Aufnahme von Gewebeproben bildet. Wird das distale Ende 17 in Gewebe gestochen und/oder dieses durch Vor- und Zurückbewegung abgeschilfert, so verbleibt eine Gewebeprobe in der distalen Hohlkammer 19 und kann mit dem Biopsieelement 15 entnommen werden. Beispielsweise kann über einen die Hohlkammer 19 ausfüllenden, distal abgeschrägten Zylinder, der durch die Bedienperson von proximaler Seite aus vor- bzw. zurückbewegt werden kann, und auf diese Weise die Hohlkammer kontrolliert blockiert bzw. freigibt, von der Bedienperson bestimmt werden, von welchem axialen Punkt im Gewebe eine Gewebeprobe entnommen wird. Das vorausgehend beschriebene Biopsieelement 15 und dessen Funktionsweise stehen beispielhaft für eine mögliche Vorgehensweise der Gewebeentnahme. Andere Formen der Gewebeentnahme, z.B. über lateral angebrachte Einkerbungen am distalen Ende des Biopsieelements 15, sind gleichfalls möglich.

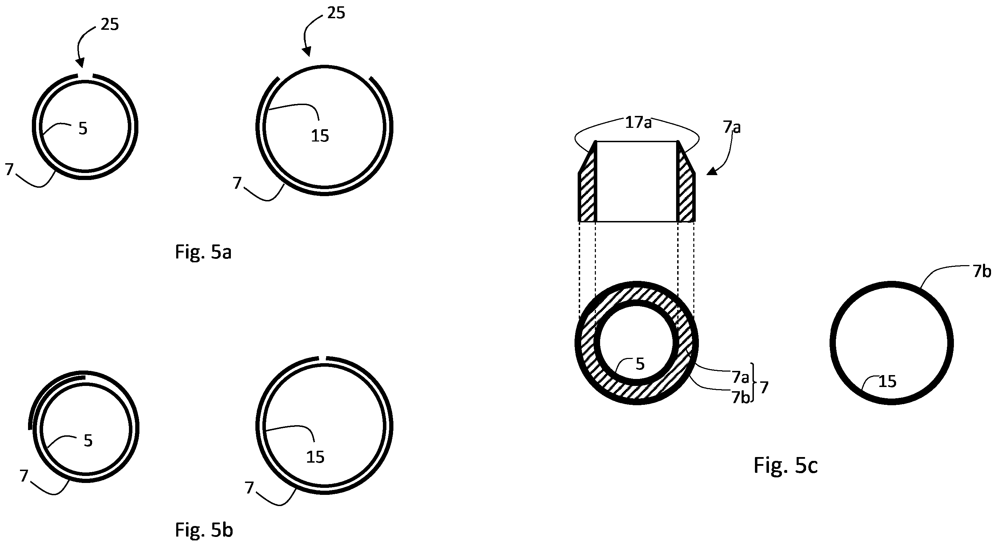

Die Einführhülse 7 hat nun den Effekt, dass das Biopsieelement 15 genau an die Stelle positioniert werden kann, an der das Fluoreszenz-Endoskopieelement ein auffälliges Fluoreszenzsignal detektiert hat. Außerdem hilft sie dem Biopsieelement 15 beim Einstich durch das Hautgewebe 9. Der Einstich des Biopsieelements 15 ist dadurch wesentlich weniger traumatisch. Wie in den Figuren 5a-c gezeigt, kann das Biopsieelement 15 durchaus wesentlich dicker sein als der distale Abschnitt 5, um eine für die pathologische Untersuchung ausreichend große Gewebeprobe entnehmen zu können. In diesem Fall kann sich die Einführhülse 7 vorzugsweise radial aufweiten, um das Biopsieelement 15 aufnehmen zu können. Eine radiale Aufweitung ist dabei weniger traumatisch als der direkte Einstich des Biopsieelements 15. Die abgelesene oder markierte Axialposition des distalen Abschnitts 5 relativ zur Einführhülse 7 kann nun mittels entsprechender Längenmarkierungen auf dem Biopsieelement 15 reproduziert werden.

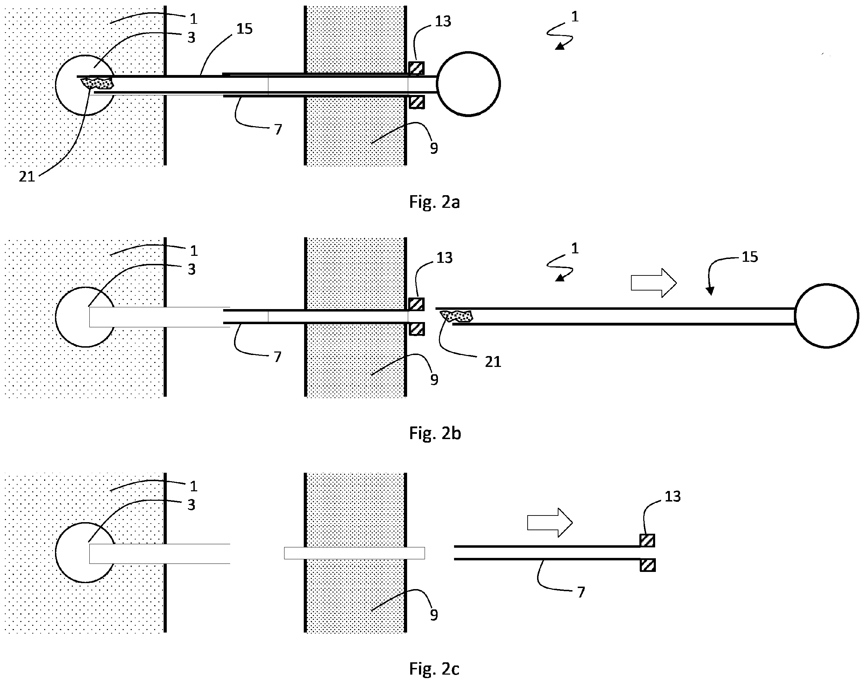

In Fig. 2a ist die reproduzierte Axialposition des Biopsieelements 15 gezeigt. Das distale Ende 17 des Biopsieelements 15 ragt um die gleiche Länge über das distale Ende der Einführhülse hinaus in das zu untersuchende Gewebe 3 im Organ 1. Dadurch wird in der distalen Hohlkammer 19, sobald diese durch entsprechende axiale Bewegung des massiven Zylinders (nicht gezeigt) in der Hohlkammer 19 in proximale Richtung durch die Bedienperson freigegeben wird, eine Gewebeprobe 21 des zu untersuchenden Gewebes 3 eingefangen. In Fig 2b ist gezeigt, wie das Biopsieelement 15 mit der Gewebeprobe 21 wieder proximal aus der Einführhülse 7 gezogen ist. Die entnommene Gewebeprobe 21 kann nun ex situ pathologisch untersucht werden. Schließlich wird, wie in Fig. 2c gezeigt, die Einführhülse 7 wieder proximalwärts aus dem Körper des Patienten gezogen.

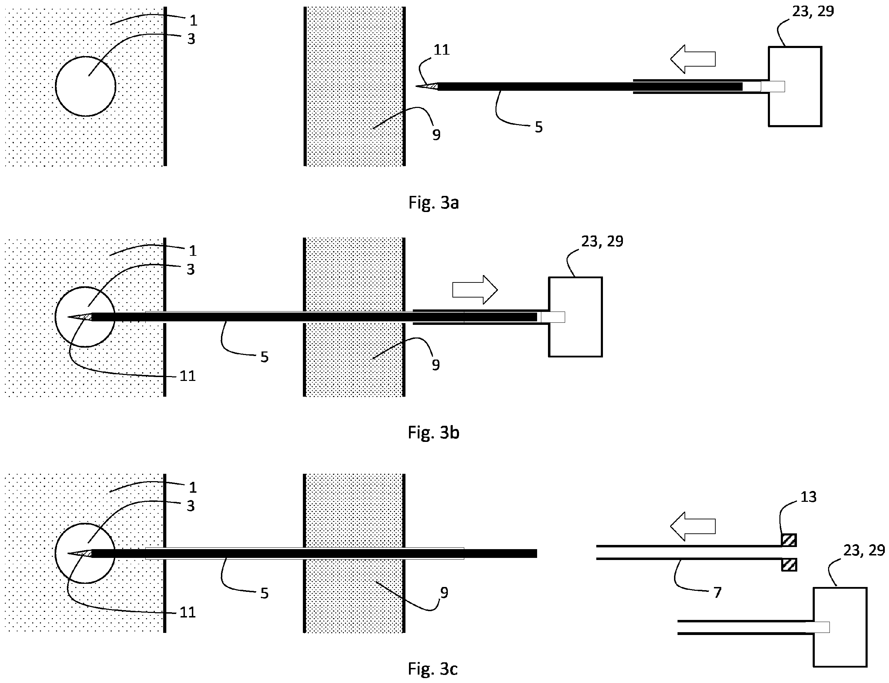

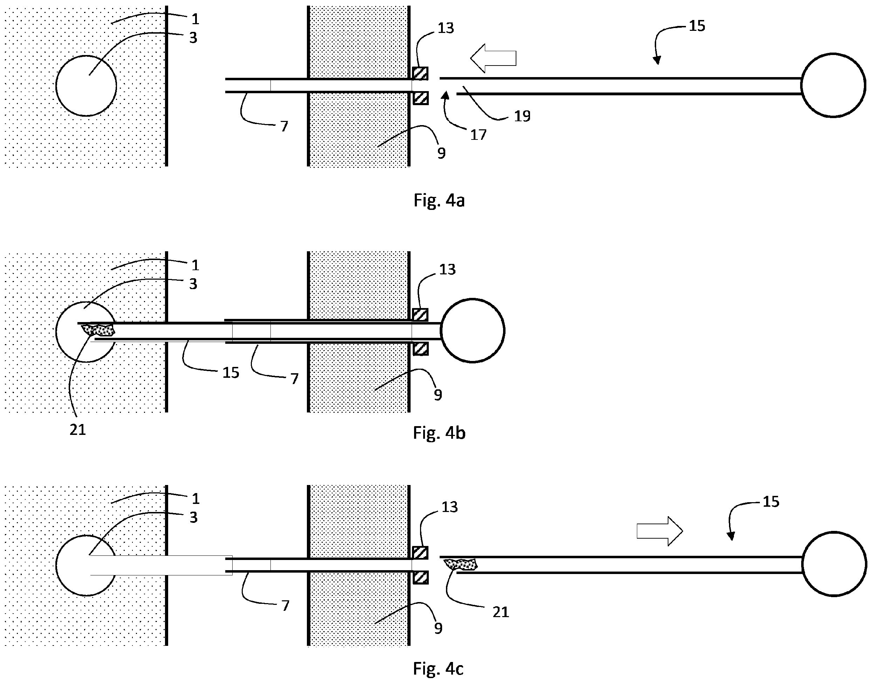

In dem in Fig. 3a-c und Fig. 4a-c gezeigten Ausführungsbeispiel ist der distale Abschnitt 5 im Gegensatz zum zuvor beschriebenen Ausführungsbeispiel biegesteifer, beispielsweise durch ein dünnwandiges Rohr versteift. Dadurch benötigt der distale Abschnitt 5 die Einführhülse 7 nicht als Einstichhilfe durch das feste Hautgewebe 9, sondern ist selbst biegesteif genug dafür. Eine an- und abkoppelbare Handhabungseinrichtung 23 kann wie hier vorgesehen sein, um die manuelle Handhabung, Positionierung und Ausrichtung des distalen Abschnitts 5 zu gewährleisten. Vorzugsweise ist eine optische Komponente 29 zur Einkopplung von Fluoreszenzlicht als Teil des Fluoreszenz-Endoskopieelements in die Handhabungseinrichtung 23 integriert.

Die Einführhülse 7 kommt hier erst zum Einsatz, wenn tatsächlich mittels des Fluoreszenzsignals eine finale Position des distalen Abschnitts 5 mit seiner distalen Spitze 11 mitten in dem zu untersuchenden Gewebe 3 gefunden und eingestellt ist. Die Handhabungseinrichtung 23 bzw. die optische Komponente 29 kann dann abgekoppelt werden (s. Fig. 3b), damit von proximal die Einführhülse 7 über den distalen Abschnitt 5 geschoben werden kann (s. Fig. 3c). Sobald die Einführhülse 7 mit dem Anschlag 13 an der Haut 3 anliegt und somit final positioniert ist, kann der distale Abschnitt 5 proximal herausgezogen werden und, wie in Figuren 4a-c gezeigt, die eigentliche Biopsie mit dem Biopsieelement 15 wie zuvor beschrieben durchgeführt werden. Die zuletzt beschriebene Vorgehensweise, d.h. die Vorgehensweise wie in den Fig. 3a-c und 4a-c dargestellt, hat gegenüber der in den Fig. 1a-c und 2a-c beschriebenen Vorgehensweise den Vorteil, dass man erstens schneller und zweitens mit geringerer Patientenbelastung (d.h. weniger traumatisch) auffälliges Gewebe aufspüren kann, da für diesen Vorgang die Einführhülse 7 noch nicht eingesetzt wird. Dieser Vorteil kommt insbesondere dann zum Tragen, wenn zur Detektion auffälliger Fluoreszenz der distale Abschnitt 5 an mehreren unterschiedlichen Stellen in der Haut 9 eingestochen werden muss.

Fig. 5a-c zeigen drei Ausführungsformen der Einführhülse 7. In den Figuren 5a-c ist jeweils links die Einführhülse 7 mit eingeführtem distalen Abschnitt 5 gezeigt und jeweils rechts die Einführhülse 7 mit eingeführtem Biopsieelement 15. Das Biopsieelement 15 ist hier jeweils dicker als der distale Abschnitt 5, hat also eine größere radiale Ausdehnung. In den Ausführungsformen gemäß Fig. 5a,b ist die Einführhülse 7 radial aufweitbar und geschlitzt. In der ersten Ausführungsform gemäß Fig. 5a weitet sich ein Schlitz 25, der sich im Wesentlichen über die gesamte Länge der Einführhülse 7 erstreckt auf, wenn das dickere Biopsieelement 15 eingeführt ist und zieht sich elastisch zusammen, wenn der dünnere distale Abschnitt 5 oder nichts eingeführt ist.

In der in Fig. 5b gezeigten zweiten Ausführungsform ist die Einführhülse 7 in Umfangsrichtung überlappend ausgeführt, wenn der dünnere distale Abschnitt 5 oder nichts eingeführt ist. Die Überlappung ist bei eingeführtem Biopsieelement 15 geringer oder, wie in Fig 5b rechts gezeigt, nicht mehr vorhanden. Wenn der Unterschied der radialen Ausdehnungen zwischen distalem Abschnitt 5 und Biopsieelement 15 groß genug ist, kann es auch sein, dass die Einführhülse 7 mit eingeführtem distalen Abschnitt 5 überlappend ausgeführt ist wie in Fig. 5b links dargestellt und bei eingeführtem Biopsieelement 15 aufgeweitet mit aufgeweitetem Schlitz 25 wie in Fig. 5a rechts dargestellt.

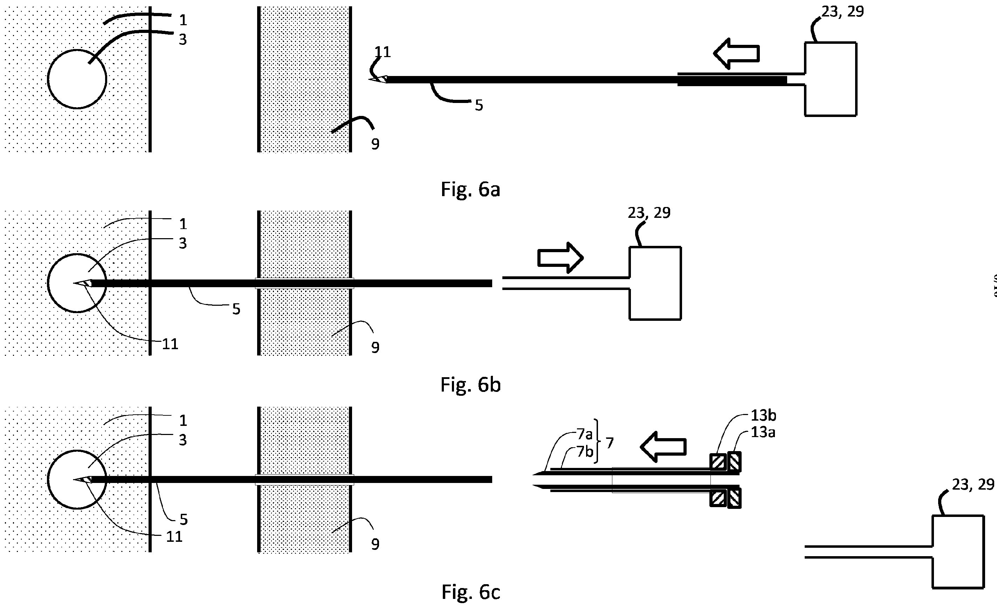

In der in Fig. 5c gezeigten dritten Ausführungsform weist die Einführhülse 7 mindestens zwei separate Teile auf, nämlich eine innenhülse 7a und eine Außenhülse 7b, die die ineinander steckbar, lösbar voneinander und gegeneinander verschiebbar sind (siehe auch Fig. 6a-c, 7a,b, 8a-c und 9). Solange noch der dünnere distale Abschnitt 5 in den Körper eingeführt ist, ist auch die Innenhülse 7a in die Außenhülse 7b eingeführt, um auf diese Weise den distalen Abschnitt 5 zu führen, d.h. ihm in radiale Richtung Halt zu geben. Um das dickere Biopsieelement 15 einzuführen und den dafür notwendigen Platz in radialer Richtung zu schaffen, wird die Innenhülse 7a aus der Außenhülse 7b proximalwärts herausgezogen, wobei die Außenhülse 7b an Ort und Stelle verbleibt. Das anschließend eingeführte, dickere Biopsieelement 15 wird dann von der Außenhülse 7b geführt, d.h. es erhält von diesem Halt in radialer Richtung. Dabei ist die Wandstärke der Innenhülse 7a so ausgeführt, dass sie im Wesentlichen der Differenz zwischen dem Außendurchmesser des Biopsieelements 15 und dem Außendurchmesser des distalen Abschnitts 5 entspricht.

Vorzugsweise hat die Innenhülse 7a distal ein konusförmiges Endstück 17a, um ein weitgehend atraumatisches Einführen in das Gewebe zu ermöglichen. Die linke obere Abbildung der Fig. 5c zeigt einen Längsschnitt durch das distale Ende der Innenhülse 7a.

Die dritte Ausführungsform hat gegenüber den beiden vorherigen Ausführungsformen den Vorteil, dass nahezu beliebig große Unterschiede zwischen distalem Abschnitt 5 und Biopsieelement 15 hinsichtlich ihrer radialen Ausdehnungen gehandelt werden können, indem einfach die Wandstärke der Innenhülse 7a entsprechend dick, d.h. an den Durchmesserunterschied angepasst, und mit entsprechend spitzem und in diesem Sinne entsprechend atraumatischem Endstück 17a ausgeführt wird. Dagegen ist die Aufweitung der Einführhülse 7 bei den vorherigen Ausführungsformen wegen der begrenzten Elastizität der Einführhülse 7 eingeschränkt. Dadurch kann der Vorteil des hier beschriebenen Biopsiesystems, viele Läsionen mit möglichst wenig Gewebeschaden zu finden und zu biopsieren, in bestmöglicher Weise umgesetzt werden. Mit einem sehr dünnen distalen Abschnitt 5 kann ausgiebig und engmaschig und dennoch patientenschonend nach auffälliger Fluoreszenz gesucht werden, weil ein sehr dünner distaler Abschnitt 5 nur wenig Schaden im gesunden Gewebe verursacht. Anschließend kann mit einem vergleichsweise dicken Biopsieelement 15, das nur noch an den Ort der auffälligen Fluoreszenz gebracht werden muss, eine relativ große und damit für den Pathologen wertvolle Gewebeprobe gewonnen werden.

Die dritte Ausführungsform hat gegenüber den beiden vorherigen Ausführungsformen den weiteren Vorteil, dass beim Einführen des Biopsieelements 15 keine Kraft zur Weitung der Einführhülse 7 aufgebracht werden muss und auch keine Reibungskraft gegenüber der in den vorherigen Ausführungsformen anliegenden Einführhülse 7 beim Vorschieben des Biopsieelements überwunden werden muss.

Wie im zweiten Ausführungsbeispiel gemäß Fig. 3a-c und 4a-c ist auch in dem in den Fig. 6a-c, 7a,b, 8a-c und 9 gezeigten dritten Ausführungsbeispiel der distale Abschnitt 5 durch ein dünnwandiges Rohr versteift, sodass keine Einstichhilfe durch das feste Hautgewebe 9 benötigt wird. Auch in diesem dritten Ausführungsbeispiel ist die optische Komponente 29 vorzugsweise so ausgeführt, dass es die Aufgaben der Handhabungseinrichtung 23 aus den Fig. 3a-c gleich mit übernimmt und eine separate zusätzliche Handhabungseinrichtung 23 nicht benötigt wird. Die Einführhülse 7, hier als Doppelhülse ausgeführt, bestehend aus Außenhülse 7b und Innenhülse 7a, kommt auch hier erst dann zum Einsatz, wenn tatsächlich die finale Position des distalen Abschnitts 5 erreicht ist, siehe Fig. 6c.

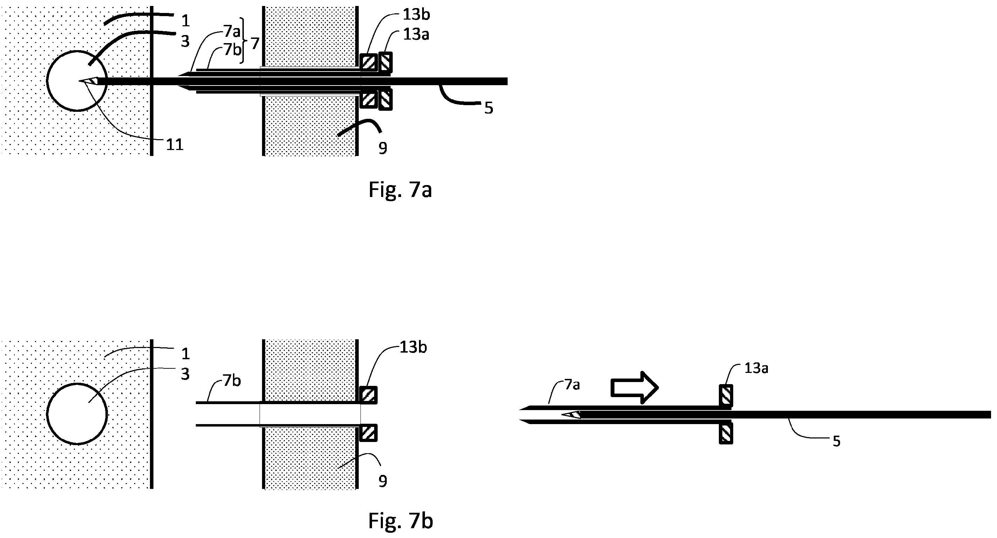

Zunächst werden beide Hülsen 7a und 7b in Position gebracht, d.h. soweit in das Gewebe eingeführt, bis ihre Anschläge 13a und 13b am Hautgewebe anliegen, siehe Fig. 7a. Anschließend wird der distale Abschnitt 5 zusammen mit der Innenhülse 7a aus dem menschlichen Körper, d.h. aus der Außenhülse 7b herausgezogen, siehe Fig. 7b, um den Zugang für das dickere Biopsieelement 15 zu schaffen.

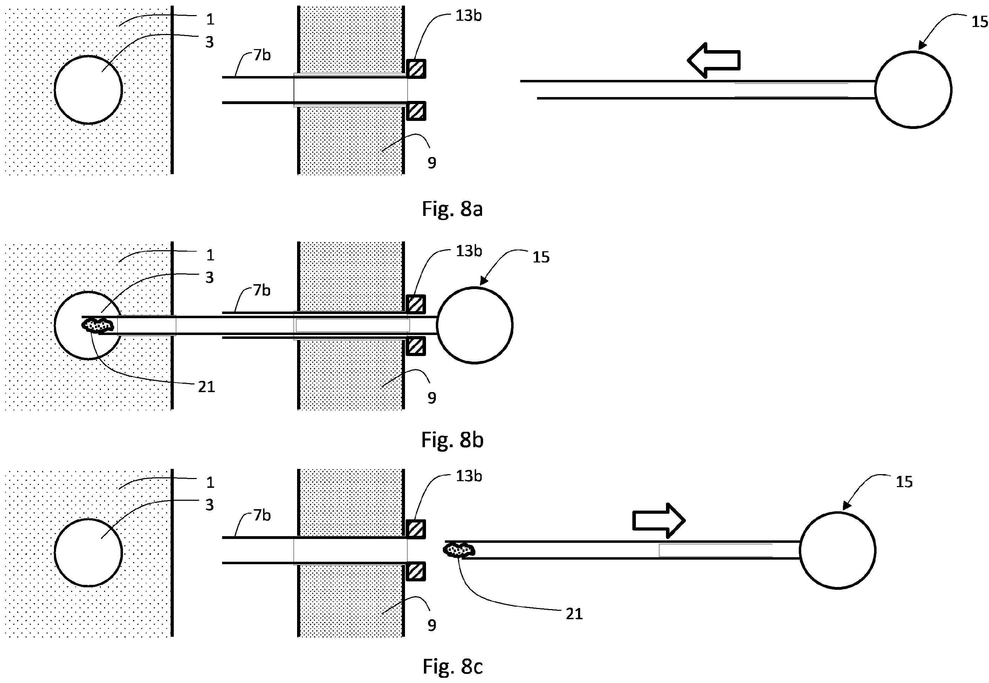

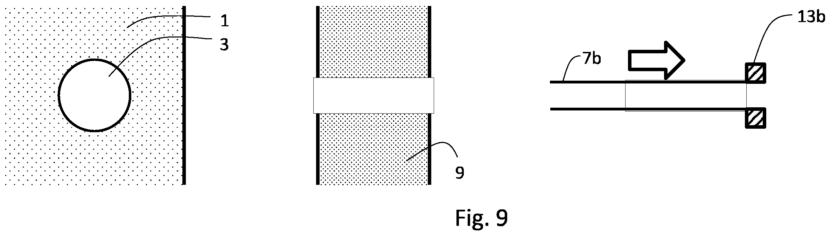

In das so bereitgestellte Lumen kann dann das dickere Biospieelement 15 eingeführt werden (siehe Fig. 8a), um schließlich eine Gewebeprobe 21 zu entnehmen (siehe Fig. 8b). Anschließend wird das Biopsieelement 15 zusammen mit der Gewebeprobe 21 aus dem Körper bzw. der Außenhülse 7b herausgenommen, um sie dem Pathologen zuzuführen (siehe Fig. 8c). Zuletzt wird noch die Außenhülse 7b entfernt (siehe Fig. 9).

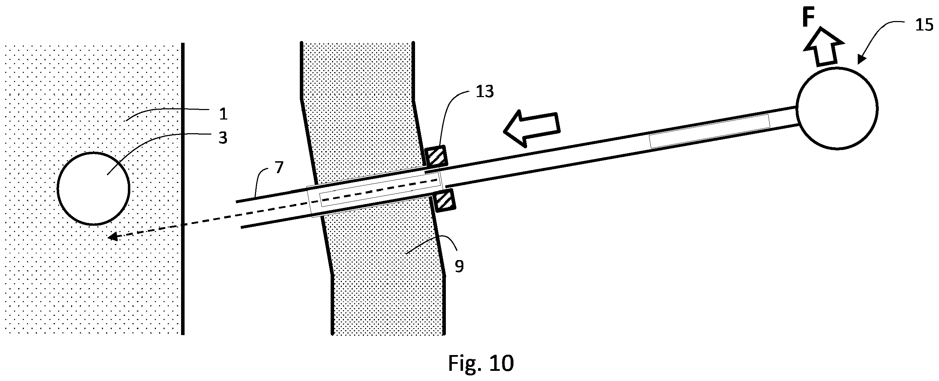

In Fig. 10 ist eine Problematik gezeigt, die bei den zuvor beschriebenen drei Ausführungsformen des Biopsiesystems vorkommen kann. Die Haut 9 kann ggf. an einer Stelle des Körpers des Patienten so flexibel sein, sodass seitlich wirkende nicht-axiale Kräfte F Hebelkräfte auf die Einführhülse 7 ausüben, welche die Ausrichtung und/oder Position der Einführhülse 7 relativ zu dem zu untersuchenden Gewebe 3 verändern können. Wenn die Einführhülse 7 allerdings die korrekte die Ausrichtung und/oder Position relativ zu dem zu untersuchenden Gewebe 3 nicht behält, besteht das Risiko, dass das Biopsieelement 15 an dem zu untersuchenden Gewebe 3 vorbeisticht. Dies ist in Fig. 10 gezeigt.

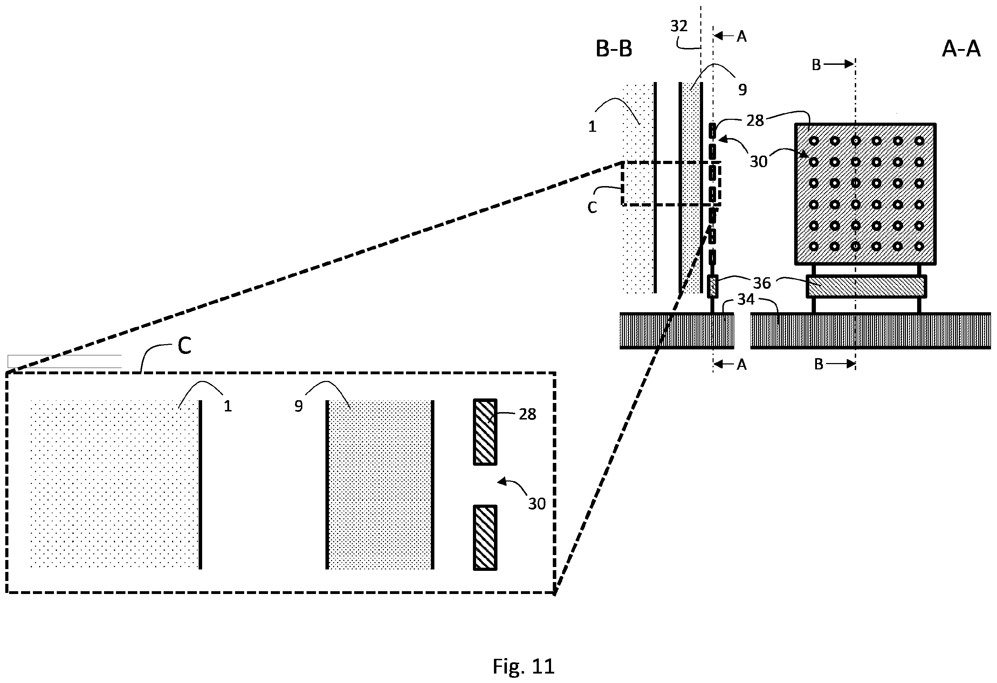

Fig. 11, 12a-c, 13a-c, 14a,b und 15a,b zeigen verschiedene Versionen einer vierten Ausführungsform des Biopsiesystems, das ferner eine Schablone 28 aufweist. Die Schablone 28 ist hier als eine Lochplatte mit einer Vielzahl an rasterartig verteilten Durchbrechungen 30 gezeigt. Die Schablone 28 ist vorzugsweise starr, zumindest wesentlich fester als die Haut 9 und relativ zum Körper 32 des Patienten fixiert. Sie kann dazu auf die Haut 9 aufgeklebt sein und/oder, wie in Fig. 11 gezeigt, auf einem Operationstisch 34 fixiert sein, auf dem der Körper 32 des Patienten eine definierte Position hat. Die Schablone 28 steht vorzugsweise flächig in direktem Kontakt mit der Haut 9 des Körpers 32 des Patienten. Die Schablone 28 ist hier über eine Positioniereinheit 36 in vorzugsweise drei verschiedenen Raumrichtungen translatorisch und/oder rotatorisch, also mit sechs Freiheitsgraden positionierbar und in einer gewählten Position und Ausrichtung relativ zum Operationstisch 34 fixierbar.

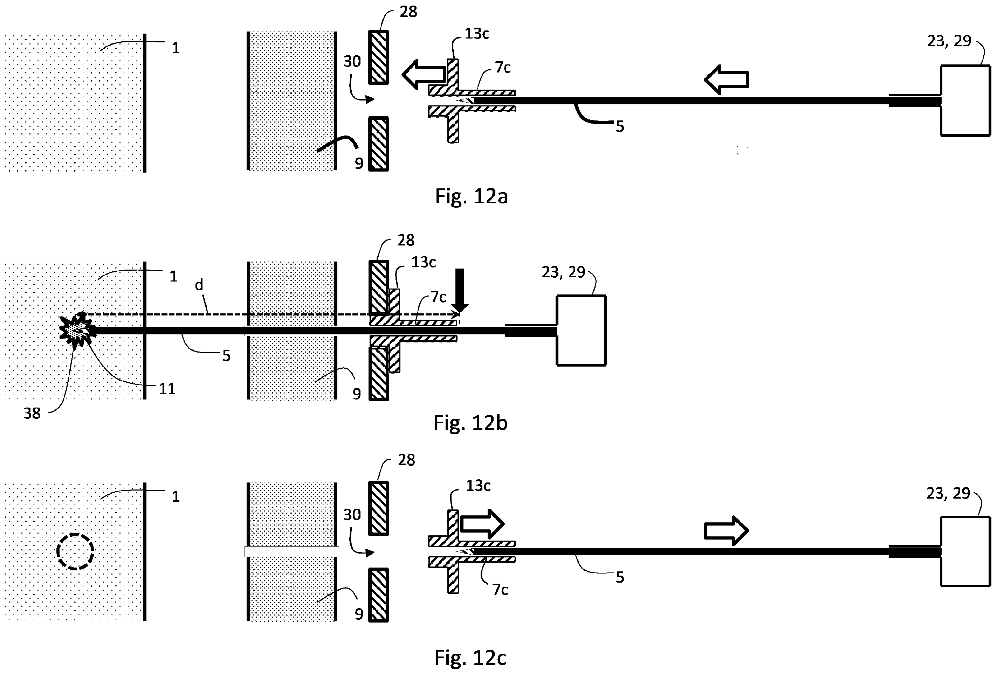

In Fig. 12a-c ist gezeigt, wie auffällig fluoreszierendes Gewebe 3 im Organ 1 mit dem Fluoreszenz-Endoskopieelement 27, das den distalen Abschnitt 5 und eine optische Komponente 29 inklusive Handhabungseinrichtung 23 aufweist, gefunden wird. Die Bedienperson ist nun nicht mehr vollkommen frei in der Entscheidung über die Einstichposition und den Einstichwinkel, sondern wählt eine Durchbrechung 30 in der Schablone 28 zum Einstich für den distalen Abschnitt 5 aus. Durch die gewählte Durchbrechung 30 ist die Einstichposition und der Einstichwinkel vollkommen festgelegt und die Bedienperson hat mit der Einstichtiefe nur noch einen Bewegungsfreiheitsgrad. Da die Durchbrechungen 30 in der Schablone 28 so groß sein müssen, dass auch das dickere Biopsieelement 15 später hindurch passt, fungiert die Einführhülse 7 hier als Adapter zwischen distalem Abschnitt 5 bzw. Biopsieelement 15 und Schablone 28. Die Einführhülse 7 wird hier nicht mehr in die Haut 9 des Patienten gestochen, was den Patienten zusätzlich schont.

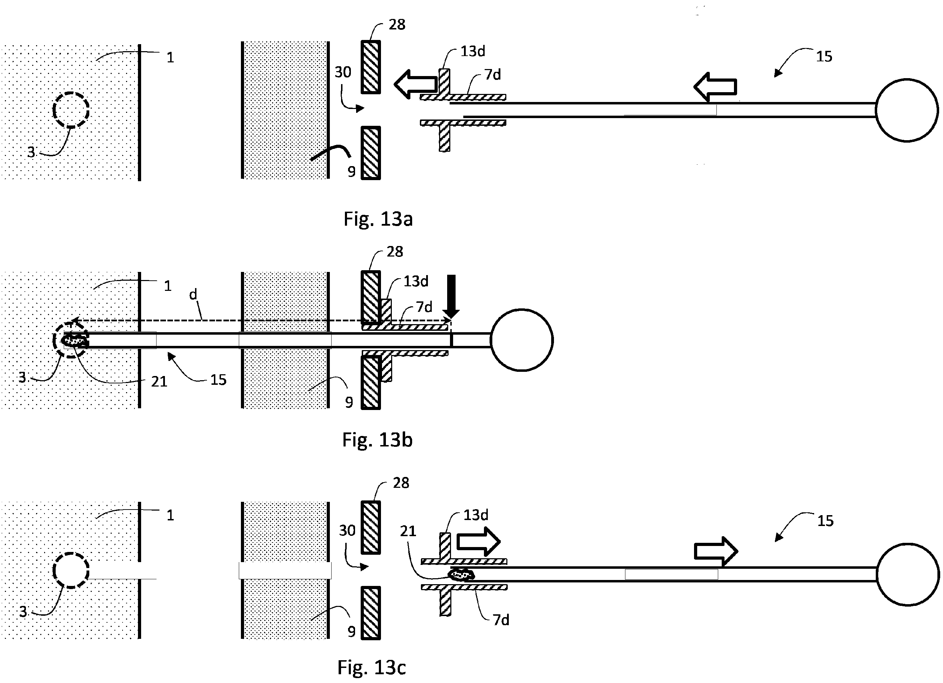

Die Einführhülse 7 weist in dieser Variante zwei Teile auf, wobei ein erster Teil 7c als Adapter zwischen distalem Abschnitt 5 und Schablone 28 und ein zweiter Teil 7d (siehe Fig. 13 a-c) als Adapter zwischen Biopsieelement 15 und Schablone 28 dient. Der erste Einführhülsenteil 7c fixiert den distalen Abschnitt 5 lateral in der gewählten Durchbrechung 30 in der Schablone 28 und liegt mit einem als Anschlag 13c fungierenden Flansch an der Schablone 28 an. Es können auch Verschlussmittel wie etwa ein Bajonettverschluss oder Luer-Anschluss für einen temporären Form- und/oder Kraftschluss zwischen dem ersten Einführhülsenteil 7c und der Schablone 28 vorgesehen sein.

Der distale Abschnitt 5 wird wie in Fig. 12b gezeigt, durch den Einführhülsenteil 7c sowie durch die Haut 9 in das zu untersuchende Organ 1 gestochen, um dort in unterschiedlichen Einstichtiefen nach einer auffälligen Fluoreszenz 38 zu suchen. Ist nun bei einer Einstichtiefe d (gemessen am proximalen Ende des ersten Einführhülsenteils 7c) eine auffällige Fluoreszenz 38 gefunden, so kann die Einstichtiefe d anhand einer Längenmarkierung am distalen Abschnitt 5 abgelesen und gemerkt werden. Alternativ oder zusätzlich kann ein verschiebbares Markierungselement an das proximale Ende des ersten Einführhülsenteils 7c geschoben und axial am distalen Abschnitt 5 fixiert werden. Sodann wird, wie in Fig. 12c gezeigt, der distale Abschnitt 5 nebst erstem Einführhülsenteil 7c aus der Schablone 28 proximalwärts herausgezogen.

Die eigentliche Biopsie ist in Fig. 13a-c gezeigt. In dieselbe Durchbrechung, in der zuvor der distale Abschnitt 5 war, wird nun das Biopsieelement 15 eingeführt, wobei ein zweiter Einführhülsenteil 7d als Adapter zwischen Biopsieelement 15 und Schablone 28 fungiert. Der zweite Einführhülsenteil 7d hat hier den gleichen an die Durchbrechung angepassten Außendurchmesser wie der erste Einführhülsenteil 7c. Da das Biopsieelement 15 allerdings dicker als der distale Abschnitt 5 ist, ist der Innendurchmesser des zweiten Einführhülsenteils 7d entsprechend größer als der Innendurchmesser des ersten Einführhülsenteils 7c. Die axiale Länge ist bei dem ersten Einführhülsenteil 7c und dem zweiten Einführhülsenteil 7d gleich. Das Biopsieelement 15 kann nun um die gemerkte Einstichtiefe d in das Organ 1 eingestochen werden, um im Bereich der auffälligen Fluoreszenz 38 aus dem zu untersuchenden Gewebe 3 eine Gewebeprobe 21 zu entnehmen.

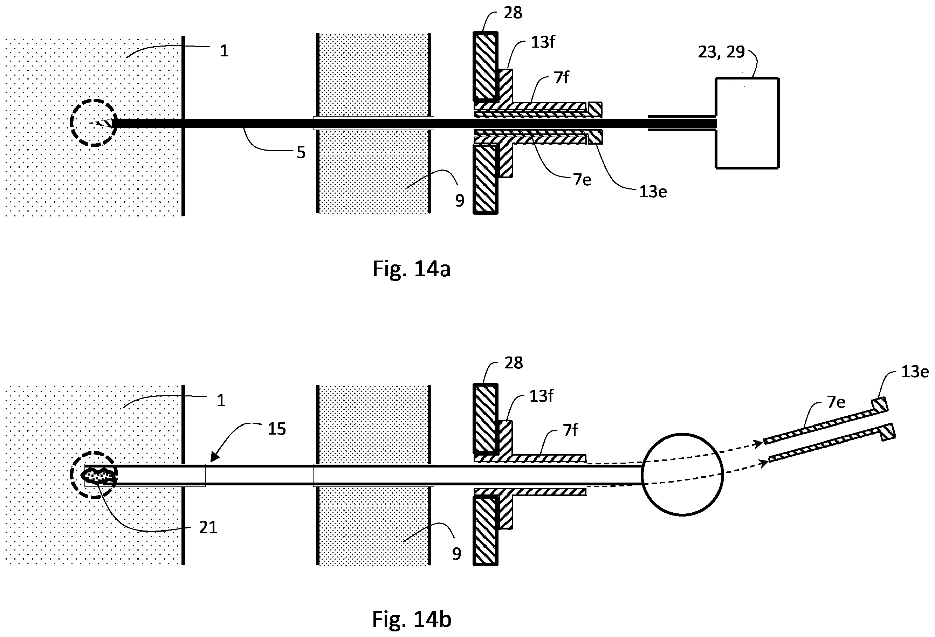

Eine zweite Variante der vierten Ausführungsform des Biopsiesystems ist in Fig. 14a,b dargestellt, wobei Fig. 14a die Lokalisierung mittels des Fluoreszenz-Endoskopieelements 27 und Fig. 14b die Biopsie mittels des Biopsieelements 15 zeigt. Im Gegensatz zur zuvor beschriebenen Variante gemäß Fig. 12a-c und 13a-c sind hier die beiden Teile der Einführhülse 7 als Innenhülse 7e und Außenhülse 7f ausgestaltet. Der Außendurchmesser der Innenhülse 7e ist an den Innendurchmesser der Außenhülse 7f sowie an den Außendurchmesser des Biopsieelements 15 angepasst. Der Innendurchmesser der Innenhülse 7e ist an den Außendurchmesser des distalen Abschnitts 5 angepasst. Der Außendurchmesser der Außenhülse 7f ist an den Innendurchmesser der Durchbrechungen 30 in der Schablone 28 angepasst. Ineinandergesteckt fungieren die Innenhülse 7e und die Außenhülse 7f als passender Adapter zwischen distalem Abschnitt 5 und Schablone 28. Die Außenhülse 7f allein bei proximalwärts herausgezogener Innenhülse 7f dient als passender Adapter zwischen Biopsieelement 15 und Schablone 28.

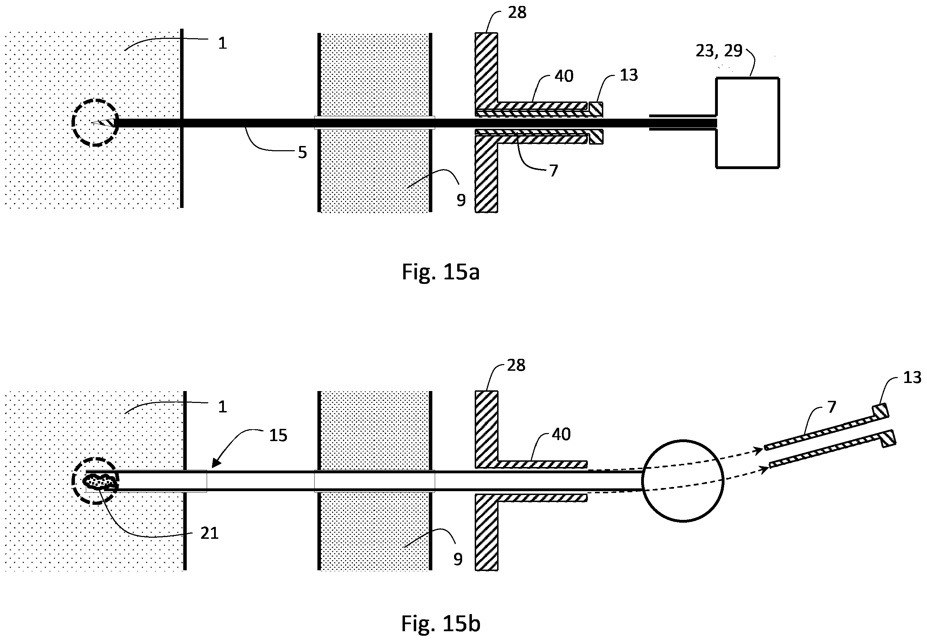

Eine dritte Variante der vierten Ausführungsform des Biopsiesystems ist in Fig. 15a,b dargestellt, wobei Fig. 15a die Lokalisierung mittels des Fluoreszenz-Endoskopieelements 27 und Fig. 15b die Biopsie mittels des Biopsieelements 15 zeigt. Hierbei ist die Einführhülse 7 einteilig ausgestaltet bzw. besteht nur aus einer Innenhülse, die als Adapter zwischen distalem Abschnitt 5 und Schablone 28 dient. Eine separate Außenhülse zur Führung des Biopsieelements 15 benötigt man hier nicht, da der Innendurchmesser der Durchbrechungen 30 bereits an den Außendurchmesser des Biopsieelements 15 angepasst ist und die Schablone 28 in proximaler Verlängerung um die Durchbrechungen 30 herum ein Führungselement 40 für das Biopsieelement 15 aufweist. Das Führungselement 40 kann alternativ bei einer gewissen axialen Dicke der Schablone 28 durch die Durchbrechung 30 selbst gebildet sein.

Wenn das Rastermaß im Hinblick auf die Eindringtiefe des Fluoreszenzanregungslichtes genügend klein ausgeführt ist, d.h. wenn die Durchbrechungen 30 genügend eng nebeneinander liegen, so dass bei sequentiell in die Durchbrechungen 30 eingeführtem distalen Abschnitt 5 jedes Volumenelement des zu untersuchenden Organs mit Fluoreszenzanregungslicht erreicht wird und wenn dann außerdem bei einer Untersuchung tatsächlich auch jede Durchbrechung 30 über die gesamte Organtiefe genutzt wird, dann ist auch sichergestellt, dass jedes Volumenelement des zu untersuchenden Organs fluoreszenztechnisch voruntersucht ist, also kein Volumenelement unberücksichtigt bzw. vergessen wurde.

Fig. 16 zeigt das Fluoreszenz-Endoskopieelement 27 schematisch genauer. Das Fluoreszenz-Endoskopieelement 27 weist den distale Abschnitt 5, eine optische Komponente 29 und eine Betriebseinheit 31 auf, wobei die optische Komponente 29 vorzugsweise die Funktion einer zuvor beschriebenen Handhabungseinrichtung 23 übernimmt. Der distale Abschnitt 5 ist proximalseitig mit der optischen Komponente 29 koppelbar oder fest gekoppelt, wodurch eine proximalwärtige Auskopplung von Fluoreszenzlicht aus dem Lichtleiter des distalen Abschnitts 5 in die optische Komponente 29 sowie eine distalwärtige Einkopplung von Anregungslicht aus der optischen Komponente 29 in den Lichtleiter erzielt wird. Die distale Spitze 11 des distalen Abschnitts 5 kann als transparente Nadelspitze ausgeführt sein, die als Auskopplung von Anregungslicht aus dem Lichtleiter und Einkopplung von Fluoreszenzlicht in den Lichtleiter dient. Zur Verstärkung der Spitze 11 und zur Reduzierung der Gewebetraumatisierung durch den Einstich des distalen Abschnitts 5 kann die Spitze 11 wie gezeigt mit einem Dorn 33 versehen, sein, der über die distale Nadelspitze hinausragen kann.

Die optische Komponente 29 weist einen Strahlteiler 35 in Form eines Strahlteilerblocks auf, der durch ein Prismenpaar gebildet wird. Die Prismen liegen jeweils mit optischen Grenzflächen aneinander, die sich in einem Winkel von ca. 45° zur optischen Achse erstrecken. Zwischen oder mindestens an oder auf einer der optischen Grenzflächen ist ein Langpassfilter 37 angeordnet, das langwelligeres Fluoreszenzlicht im Wesentlichen transmittiert und kurzwelliges Anregungslicht reflektiert. Im Wesentlichen koaxial zur optischen Achse weist der Strahlteiler 35 daher eine Bildpfadseite auf, an der ein Bildsensor 39, eine Photodiode o.ä. angeordnet ist. Lateral weist der Strahlteiler 35 eine Beleuchtungspfadseite auf, an welcher eine Lichtquelle 41, beispielsweise in Form einer LED oder einer Laserauskopplung, angeordnet ist. Das Langpassfilter 37 reflektiert das Anregungslicht distalwärts zum distale Abschnitt 5. Vorzugsweise ist die optische Komponente 29 in einen Handgriff oder in eine ähnliche ergonomische Komponente integriert, welche am distale Abschnitt 5 sicher und dennoch lösbar arretiert werden kann, so dass sie die Aufgaben/Funktionen der Handhabungseinrichtung 23 mitübernehmen kann. Dadurch wird es möglich, erstens den gesamten Bewegungsvorgang des distalen Abschnitts 5 im Gewebe im Hinblick auf die Suche nach pathologisch relevantem Gewebe fluoreszenzoptisch zu überwachen und zweitens auf die Handhabungseinrichtung 23 als solche und einen Tausch derselben mit der optischen Komponente 29 zu verzichten.

Das Fluoreszenz-Endoskopieelement 27 ist vorzugsweise dazu eingerichtet, wahlweise ein erstes Anregungslicht und ein zweites Anregungslicht proximal einzukoppeln, wobei das erste Anregungslicht im Mittel kurzwelliger ist als das zweite Anregungslicht. Außerdem ist das Fluoreszenz-Endoskopieelement 27 vorzugsweise dazu eingerichtet, wahlweise ein erstes Bild bzw. ein erstes Fluoreszenzsignal von einem ersten Gewebebereich aus mit dem ersten Anregungslicht angeregtem ersten Fluoreszenzlicht zu erzeugen und ein zweites Bild bzw. ein zweites Fluoreszenzsignal von einem zweiten Gewebebereich aus mit dem zweiten Anregungslicht angeregtem zweiten Fluoreszenzlicht aufzunehmen, wobei das erste Bild bzw. das erste Fluoreszenzsignal aus einem kleineren Gewebevolumen stammt als das zweite Bild bzw. das zweite Fluoreszenzsignal, aber dadurch eine höhere räumliche Auflösung aufweist.

Die Betriebseinheit 31 ist mit dem Bildsensor 39, der Photodiode o.ä. und der Lichtquelle 41 über eine Kabelverbindung 43 signalverbunden oder -verbindbar. Die Lichtquelle 41 kann über die Betriebseinheit 31 gesteuert und die Signale des Bildsensors 39, der Photodiode o.ä. können von der Betriebseinheit 31 empfangen, verarbeitet und visualisiert werden. Dazu weist die Betriebseinheit eine optische und/oder akustische Anzeigekomponente 45, eine Steuer- und Versorgungskomponente 47 und eine Bedienungskomponente 49 auf.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Entsprechend soll der Schutzbereich solche äquivalente Ausführungsformen umfassen, solange sie unter den Umfang des Anspruchs 1 fallen. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, vom Schutzbereich dieser Offenbarung mit erfasst, solange sie unter den Umfang des Anspruchs 1 fallen. Im Übrigen soll hierin weder der Begriff "aufweisen" zusätzliche andere Merkmale oder Verfahrensschritte ausschließen noch soll "ein" oder "eine" eine Mehrzahl ausschließen.

### Bezugszeichenliste:

- 1: Organ
- 3: zu untersuchendes Gewebe
- 5: distaler Abschnitt des Fluoreszenz-Endoskopieelements
- 7: Einführhülse
- 7a, f: Innenhülse
- 7b, e: Außenhülse
- 7c: erster Einführhülsenteil
- 7d: zweiter Einführhülsenteil
- 9: Haut/Hautgewebe
- 11: distale Spitze des distalen Abschnitt des Fluoreszenz-Endoskopieelements
- 13: Anschlag der Einführhülse
- 13a,e: Anschlag der Innenhülse
- 13b,f: Anschlag der Außenhülse
- 13c: Anschlag des ersten Einführhülsenteils
- 13d: Anschlag des zweiten Einführhülsenteils
- 15: Biopsieelement
- 17: distales Ende des Biopsieelements
- 17a: konusförmiges Endstück
- 19: Hohlkammer des Biopsieelements
- 21: Gewebeprobe
- 23: Handhabungseinrichtung
- 25: Schlitz
- 27: Fluoreszenz-Endoskopieelement
- 28: Schablone
- 29: optische Komponente
- 30: Durchbrechungen
- 31: Betriebseinheit
- 32: Körper des Patienten
- 33: Dorn
- 34: Operationstisch
- 35: Strahlteiler
- 36: Positioniereinheit
- 37: Langpassfilter
- 38: auffällige Fluoreszenz
- 39: Bildsensor, Photodiode, o.ä.
- 40: Führungselement
- 41: Lichtquelle
- 43: Kabelverbindung
- 45: Anzeigekomponente
- 47: Steuer- und Versorgungskomponente
- 49: Bedienungskomponente

## Patentansprüche

1. Biopsiesystem mit
- einem Fluoreszenz-Endoskopieelement (27),
- einem Biopsieelement (15) zur Entnahme einer Probe (21) des zu untersuchenden Gewebes (3) aus einem organischen Körper, und
- einer Einführhülse (7) zum Platzieren eines distalen Endes (11, 17) des Fluoreszenz-Endoskopieelements (27) und des Biopsieelements (15) in oder an das zu untersuchende Gewebe (3) im organischen Körper,
wobei das Fluoreszenz-Endoskopieelement (27) einen distalen Abschnitt (5) mit einer distalseitigen Auskopplung von Anregungslicht sowie einer distalseitigen Einkopplung von Fluoreszenzlicht aufweist,
wobei der distale Abschnitt (5) des Fluoreszenz-Endoskopieelements (27) relativ zur Einführhülse (7) axial beweglich positionierbar und proximalwärts aus der Einführhülse (7) entnehmbar ist,
wobei das Biopsieelement (15) bei entnommenem distalen Abschnitt (5) des Fluoreszenz-Endoskopieelements (27) durch die Einführhülse (7) hindurch in oder an das zu untersuchende Gewebe (3) im organischen Körper platzierbar ist, **dadurch gekennzeichnet, dass** das Biopsieelement (15) eine größere radiale Ausdehnung hat als der distale Abschnitt (5) des Fluoreszenz-Endoskopieelements (27) und ein Innendurchmesser der Einführhülse (7) zur wahlweisen Aufnahme des distalen Abschnitts (5) des Fluoreszenz-Endoskopieelements (27) und des Biopsieelements (15) anpassbar ist.

2. Biopsiesystem nach Anspruch 1, wobei die Einführhülse (7) biegesteifer als der distale Abschnitt (5) des Fluoreszenz-Endoskopieelements (27) ist.

3. Biopsiesystem nach Anspruch 1 oder 2, wobei die Einführhülse (7) proximalseitig distalwärts über den distalen Abschnitt (5) des Fluoreszenz-Endoskopieelements (27) stülpbar ist.

4. Biopsiesystem nach einem der vorhergehenden Ansprüche, wobei die Einführhülse (7) axial geschlitzt und aufweitbar ist.

5. Biopsiesystem nach Anspruch 4, wobei die Einführhülse (7) zur Aufnahme des distalen Abschnitts (5) des Fluoreszenz-Endoskopieelements (27) in Umfangsrichtung überlappend ausgeführt ist, wobei die Überlappung bei aufgenommenem Biopsieelement (15) geringer ist oder nicht mehr besteht.

6. Biopsiesystem nach einem der Ansprüche 3 bis 5, wobei die Einführhülse (7) eine Innenhülse (7a,e) und eine Außenhülse (7b,f) aufweist, wobei zur Aufnahme des Biopsieelements (15) die Innenhülse (7a,e) aus der Außenhülse (7b,f) proximalwärts entfernbar ist.

7. Biopsiesystem nach Anspruch 6 wobei die Innenhülse (7a,e) einen Innendurchmesser aufweist, der im Wesentlichen einem Außendurchmesser des distalen Abschnitts (5) des Fluoreszenz-Endoskopieelements (27) entspricht, und die Außenhülse (7b,f) einen Innendurchmesser aufweist, der im Wesentlichen einem Außendurchmesser des Biopsieelements (15) entspricht.

8. Biopsiesystem nach einem der vorhergehenden Ansprüche, wobei sowohl der distale Abschnitt (5) des Fluoreszenz-Endoskopieelements (27) als auch das Biopsieelement (15) Längenmarkierungen und/oder einen axial positionierbaren Anschlag für eine definiert reproduzierbare relative Axialposition des distalen Abschnitts (5) bzw. des Biopsieelements (15) zur Einführhülse (7) aufweisen.

9. Biopsiesystem nach einem der vorhergehenden Ansprüche, wobei das Fluoreszenz-Endoskopieelement (27) dazu eingerichtet ist, wahlweise ein erstes Anregungslicht und ein zweites Anregungslicht proximal einzukoppeln, wobei das erste Anregungslicht im Mittel kurzwelliger ist als das zweite Anregungslicht, und wobei das Fluoreszenz-Endoskopieelement (27) dazu eingerichtet ist, wahlweise ein erstes Fluoreszenzsignal von einem ersten Gewebebereich aus mit dem ersten Anregungslicht angeregtem ersten Fluoreszenzlicht zu erzeugen und ein zweites Fluoreszenzsignal von einem zweiten Gewebebereich aus mit dem zweiten Anregungslicht angeregtem zweiten Fluoreszenzlicht aufzunehmen.

10. Biopsiesystem (1) nach einem der vorhergehenden Ansprüche, wobei der distale Abschnitt (5) des Fluoreszenz-Endoskopieelements (27) einen Lichtleiter mit einer proximalseitigen Einkopplung von Anregungslicht und/oder einer proximalseitigen Auskopplung von Fluoreszenzlicht aufweist, wobei das distale Ende (11, 17) des Fluoreszenz-Endoskopieelements (27) als distalseitige Auskopplung des Anregungslichts aus dem Lichtleiter und/oder als distalseitige Einkopplung des Fluoreszenzlichts in den Lichtleiter fungiert.

11. Biopsiesystem nach Anspruch 10, wobei das Fluoreszenz-Endoskopieelement (27) eine proximalseitig mit dem distalen Abschnitt (5) des Fluoreszenz-Endoskopieelements (27) lösbar koppelbare oder fest gekoppelte optische Komponente (29) aufweist, wobei die optische Komponente (29) einen Strahlteiler (35) mit einer Bildpfadseite und einer Beleuchtungspfadseite aufweist, wobei zum Auskoppeln des Fluoreszenzlichts ein Bildsensor (39) oder eine Photodiode mit der Bildpfadseite lösbar koppelbar oder fest gekoppelt ist, und wobei zum Einkoppeln des Anregungslichts eine Lichtquelle (41) mit der Beleuchtungspfadseite lösbar koppelbar oder fest gekoppelt ist.

12. Biopsiesystem nach Anspruch 11, wobei die optische Komponente (29) ein optisches Langpassfilter (37) aufweist, welches das Fluoreszenzlicht stärker zur Bildpfadseite transmittiert als das Anregungslicht, und/oder ein optisches Kurzpassfilter aufweist, welches das Fluoreszenzlicht stärker zur Bildpfadseite reflektiert als das Anregungslicht.

13. Biopsiesystem nach einem der Ansprüche 11 oder 12, wobei das Fluoreszenz-Endoskopieelement (27) eine mit dem Bildsensor (39) oder der Photodiode und/oder der Lichtquelle (41) signalverbundene oder-verbindbare Betriebseinheit (31) aufweist, wobei die Betriebseinheit (31) eine optische oder akustische Anzeigekomponente (45), eine Steuer- und Versorgungskomponente (47) und eine Bedienungskomponente (49) aufweist.

14. Biopsiesystem nach einem der vorhergehenden Ansprüche, ferner aufweisend eine bezüglich des organischen Körpers positionierbare und fixierbare Schablone (28) mit mindestens einer Durchbrechung (30) zur selektiven Aufnahme der Einführhülse (7) und des Biopsieelements (15), wobei die Einführhülse (7) und/oder das Biopsieelement (15) in der mindestens einen Durchbrechung (30) gegen laterale Verschiebung und Verkippung durch die Schablone (30) fixiert ist.

15. Biopsiesystem nach Anspruch 14, wobei die Einführhülse (7) einen ersten Teil (7c,f) und einen zweiten Teil (7d,e) aufweist, wobei der erste Teil (7c,f) den distalen Abschnitt (5) des Fluoreszenz-Endoskopieelements (27) in der mindestens einen Durchbrechung (30) gegen laterale Verschiebung und Verkippung fixiert, und wobei der zweite Teil (7d,e) das Biopsieelement (15) in der mindestens einen Durchbrechung (30) gegen laterale Verschiebung und Verkippung fixiert.

16. Biopsiesystem nach Anspruch 14 oder 15, wobei die Schablone (28) eine Vielzahl von Durchbrechungen (30) aufweist, welche wahlweise zur Aufnahme der Einführhülse (7) und des Biopsieelements (15) zur Verfügung stehen.

17. Biopsiesystem (1) nach einem der vorhergehenden Ansprüche, wobei der distale Abschnitt (7) des Fluoreszenz-Endoskopieelements (27) distalseitig eine LED zur Auskopplung von Anregungslicht und/oder distalseitig einen Bildsensor oder eine Photodiode zur Einkopplung von Fluoreszenzlicht aufweist.

## Claims

1. A biopsy system comprising
- a fluorescence endoscopy element (27),
- a biopsy element (15) for taking, from an organic body, a sample (21) of the tissue (3) to be examined, and
- an insertion sleeve (7) for placing a distal end (11, 17) of the fluorescence endoscopy element (27) and of the biopsy element (15) in or on the tissue (3) in the organic body to be examined,
wherein the fluorescence endoscopy element (27) has a distal portion (5) where excitation light is distally coupled out and fluorescent light is distally coupled in, wherein the distal portion (5) of the fluorescence endoscopy element (27) can be axially movably positioned relative to the insertion sleeve (7) and can be removed proximally from the insertion sleeve (7),
wherein the biopsy element (15) can be placed in or on the tissue (3) in the organic body to be examined through the insertion sleeve (7) when the distal portion (5) of the fluorescence endoscopy element (27) is removed, **characterised in that** the biopsy element (15) has a larger radial extent than the distal portion (5) of the fluorescence endoscopy element (27) and an inner diameter of the insertion sleeve (7) is adjustable to selectively receive the distal portion (5) of the fluorescence endoscopy element (27) and the biopsy element (15).

2. The biopsy system according to claim 1, wherein the insertion sleeve (7) is more flexurally resistant than the distal portion (5) of the fluorescence endoscopy element (27).

3. The biopsy system according to claim 1 or 2, wherein the insertion sleeve (7) can be drawn proximally in the distal direction over the distal portion (5) of the fluorescence endoscopy element (27).

4. The biopsy system according to any one of the preceding claims, wherein the insertion sleeve (7) is axially slotted and expandable.

5. The biopsy system according to claim 4, wherein the insertion sleeve (7) is designed to receive the distal portion (5) of the fluorescence endoscopy element (27) in an overlapping manner in the circumferential direction, wherein the overlap is smaller or no longer exists when the biopsy element (15) is received.

6. The biopsy system according to any one of claims 3 to 5, wherein the insertion sleeve (7) has an inner sleeve (7a, e) and an outer sleeve (7b, f), wherein the inner sleeve (7a, e) can be removed in the proximal direction from the outer sleeve (7b, f) to receive the biopsy element (15).

7. The biopsy system according to claim 6, wherein the inner sleeve (7a, e) has an inner diameter that corresponds substantially to an outer diameter of the distal portion (5) of the fluorescence endoscopy element (27), and the outer sleeve (7b, f) has an inner diameter that corresponds substantially to an outer diameter of the biopsy element (15).

8. The biopsy system according to any one of the preceding claims, wherein both the distal portion (5) of the fluorescence endoscopy element (27) and the biopsy element (15) have longitudinal markings and/or an axially positionable stop for an axial position of the distal portion (5) or the biopsy element (15) relative to the insertion sleeve (7) that can be reproduced in a defined manner.

9. The biopsy system according to any one of the preceding claims, wherein the fluorescence endoscopy element (27) is set up to couple in proximally a first excitation light and a second excitation light selectively, wherein the first excitation light has shorter waves on average than the second excitation light, and wherein the fluorescence endoscopy element (27) is set up to generate a first fluorescence signal of a first tissue region from first fluorescence light excited with the first excitation light and to receive a second fluorescence signal of a second tissue region from second fluorescence light excited with the second excitation light.

10. The biopsy system (1) according to any one of the preceding claims, wherein the distal portion (5) of the fluorescence endoscopy element (27) has a light guide where excitation light is proximally coupled out and/or fluorescent light is proximally coupled out, wherein the distal end (11, 17) of the fluorescence endoscopy element (27) functions as a means for distally coupling out the excitation light from the light guide and/or as a means for distally coupling in the fluorescence light into the light guide.

11. The biopsy system according to claim 10, wherein the fluorescence endoscopy element (27) has an optical component (29) that can be releasably coupled or is fixedly coupled proximally to the distal portion (5) of the fluorescence endoscopy element (27), wherein the optical component (29) has a beam splitter (35) with an image path side and an illumination path side, wherein an image sensor (39) or a photodiode can be releasably coupled or is fixedly coupled to the image path side to couple out the fluorescence light, and wherein a light source (41) can be releasably coupled or is fixedly coupled to the illumination path side to couple in the excitation light.

12. The biopsy system according to claim 11, wherein the optical component (29) has an optical longpass filter (37), which transmits the fluorescence light more heavily to the image path side than the excitation light, and/or an optical shortpass filter, which reflects the fluorescence light more heavily to the image path side than the excitation light.

13. The biopsy system according to any one of claims 11 or 12, wherein the fluorescence endoscopy element (27) has an operating unit (31) that is connected or connectable to the image sensor (39) or the photodiode and/or the light source (41) for signal exchange, wherein the operating unit (31) has an optical or acoustic display component (45) a control and supply component (47) and an operating component (49).

14. The biopsy system according to any one of the preceding claims, further having a template (28), which can be positioned and can be fixed relative to the organic body and has at least one aperture (30) for selectively receiving the insertion sleeve (7) and the biopsy element (15), wherein the insertion sleeve (7) and/or the biopsy element (15) is fixed in the at least one aperture (30) against lateral displacement and tilting by the template (30).

15. The biopsy system according to claim 14, wherein the insertion sleeve (7) has a first part (7c, f) and a second part (7d, e), wherein the first part (7c, f) fixed the distal portion (5) of the fluorescence endoscopy element (27) in the at least one aperture (30) against lateral displacement and tilting, and wherein the second part (7d, e) fixes the biopsy element (15) in the at least one aperture (30) against lateral displacement and tilting.

16. The biopsy system according to claim 14 or 15, wherein the template (28) has a multiplicity of apertures (30), which are available selectively for receiving the insertion sleeve (7) and the biopsy element (15).

17. The biopsy element (1) according to any one of the preceding claims, wherein the distal portion (7) of the fluorescence endoscopy element (27) has distally an LED for coupling out excitation light and/or distally an image sensor or a photodiode for coupling in fluorescence light.

## Revendications

1. Système de biopsie comportant
- un élément d'endoscopie à fluorescence (27),
- un élément de biopsie (15) destiné à prélever un échantillon (21) du tissu à analyser (3) dans un corps organique, et
- un manchon d'insertion (7) permettant de placer une extrémité distale (11, 17) de l'élément d'endoscopie à fluorescence (27) et de l'élément de biopsie (15) dans ou au niveau du tissu à analyser (3) dans le corps organique,
l'élément d'endoscopie à fluorescence (27) comportant une section distale (5) avec un découplage distal de la lumière d'excitation et un couplage distal de la lumière fluorescente,
la section distale (5) de l'élément d'endoscopie à fluorescence (27) étant positionnable de manière mobile axialement par rapport au manchon d'insertion (7) et pouvant être retirée du manchon d'insertion (7) en direction proximale,
l'élément de biopsie (15) pouvant être placé dans ou à travers le tissu à analyser (3) du corps organique à travers le manchon d'insertion (7) lorsque la section distale (5) de l'élément d'endoscopie à fluorescence (27) est enlevée, **caractérisé en ce que** l'élément de biopsie (15) a une plus grande extension radiale que la section distale (5) de l'élément d'endoscopie à fluorescence (27) et qu'un diamètre intérieur du manchon d'insertion (7) peut être ajusté pour accueillir facultativement la section distale (5) de l'élément d'endoscopie à fluorescence (27) et de l'élément de biopsie (15).

2. Système de biopsie selon la revendication 1, dans lequel le manchon d'insertion (7) est plus résistant à la flexion que la section distale (5) de l'élément d'endoscopie à fluorescence (27).

3. Système de biopsie selon la revendication 1 ou 2, dans lequel le manchon d'insertion (7) peut être emboîté dans la direction distale sur la section distale (5) de l'élément d'endoscopie à fluorescence (27).

4. Système de biopsie selon l'une des revendication précédentes, dans lequel le manchon d'insertion (7) est fendu axialement et extensible.

5. Système de biopsie selon la revendication 4, dans lequel le manchon d'insertion (7) est conçu de manière à être en chevauchement circonférentiel pour accueillir la section distale (5) de l'élément d'endoscopie à fluorescence (27), le chevauchement étant inférieur ou inexistant lorsque l'élément de biopsie est posé (15).

6. Système de biopsie selon l'une des revendications 3 à 5, dans lequel le manchon d'insertion (7) comporte un manchon intérieur (7a,e) et un manchon extérieur (7b,f), sachant que, pour accueillir l'élément de biopsie (15), le manchon intérieur (7a,e) peut être retiré du manchon extérieur (7b,f) en direction proximale.

7. Système de biopsie selon la revendication 6, dans lequel le manchon intérieur (7a,e) présente un diamètre intérieur correspondant sensiblement à un diamètre extérieur de la section distale (5) de l'élément d'endoscopie à fluorescence (27), et la douille extérieure (7b,f) présente un diamètre intérieur qui correspond essentiellement à un diamètre extérieur de l'élément de biopsie (15).

8. Système de biopsie selon l'une des revendications précédentes, dans lequel aussi bien la section distale (5) de l'élément d'endoscopie à fluorescence (27) que l'élément de biopsie (15) présentent des marquages de longueur et/ou une butée positionnable axialement pour une position axiale relative reproductible de manière définie de la section distale (5) ou de l'élément de biopsie (15) par rapport au manchon d'insertion (7).

9. Système de biopsie selon l'une des revendications précédentes, dans lequel l'élément d'endoscopie à fluorescence (27) est configuré pour coupler au choix une première lumière d'excitation et une seconde lumière d'excitation au niveau proximal, la première lumière d'excitation est en moyenne plus courte que la seconde lumière d'excitation, et l'élément d'endoscopie à fluorescence (27) est configuré pour produire, au choix, un premier signal de fluorescence à partir d'une première zone de tissu avec la première lumière fluorescente excitée par la première lumière d'excitation, et recevoir un second signal de fluorescence à partir d'une seconde zone de tissu avec la seconde lumière fluorescente excitée par la seconde lumière d'excitation.

10. Système de biopsie (1) selon l'une des revendications précédentes, dans lequel la section distale (5) de l'élément d'endoscopie à fluorescence (27) a un conducteur optique avec couplage proximal de la lumière d'excitation et/ou couplage proximal de la lumière fluorescente ; l'extrémité distale (11, 17) de l'élément d'endoscopie à fluorescence (27) permettant le découplage distal de la lumière d'excitation provenant du conducteur de lumière et/ou le couplage distal de la lumière fluorescente dans le conducteur de lumière.

11. Système de biopsie selon la revendication 10, dans lequel l'élément d'endoscopie à fluorescence (27) présente un composant optique pouvant être couplé de manière amovible ou fixe (29) avec la section distale (5) de l'élément d'endoscopie à fluorescence (27), le composant optique (29) comporte un séparateur de faisceau (35) avec un côté parcours d'image et un côté parcours d'éclairage ; sachant que, pour découpler la lumière fluorescente, un capteur d'image (39) ou une photodiode peut être couplé de manière amovible ou fixe avec le côté parcours d'image et, pour coupler la lumière d'excitation, une source lumineuse (41) peut être couplée de manière fixe ou amovible au côté parcours d'éclairage.

12. Système de biopsie selon la revendication 11, dans lequel le composant optique (29) présente un filtre passe-long optique (37) qui transfère la lumière fluorescente plus fortement vers le côté parcours d'image que la lumière d'excitation, et/ou présente filtre passe-long optique qui réfléchit plus fortement la lumière fluorescente sur le côté parcours d'image que la lumière d'excitation.

13. Système de biopsie selon l'une des revendications 11 ou 12, dans lequel l'élément d'endoscopie à fluorescence (27) présente une unité de fonctionnement (31) reliée ou pouvant être reliée par signaux au capteur d'image (39) ou à la photodiode et/ou à la source lumineuse (41), l'unité de fonctionnement (31) comportant un composant d'affichage optique ou acoustique (45), un composant de commande et d'alimentation (47) et un composant de commande (49).

14. Système de biopsie selon l'une des revendications précédentes, en outre, présentant un gabarit (28) positionnable et fixable par rapport au corps organique, avec au moins une percée (30) pour l'inclusion sélective de la douille d'insertion (7) et de l'élément de biopsie (15), le manchon d'insertion (7) et/ou l'élément de biopsie (15) étant fixés dans au moins une percée (30) pour éviter le déplacement latéral et le basculement à travers le gabarit (30).

15. Système de biopsie selon la revendication 14, dans lequel le manchon d'insertion (7) comporte une première partie (7c,f) et une seconde partie (7d,e), la première partie (7c,f) fixant la section distale (5) de l'élément d'endoscopie à fluorescence (27) dans l'au moins une percée (30) pour éviter le déplacement latéral et le basculement ; et la seconde partie (7d,e) fixant l'élément de biopsie (15) dans l'au moins une percée (30) pour éviter le déplacement latéral et le basculement.

16. Système de biopsie selon la revendication 14 ou 15, dans lequel le gabarit (28) présente une multitude de percées (30) disponibles au choix pour accueillir le manchon d'insertion (7) et l'élément de biopsie (15).

17. Système de biopsie (1) selon l'une des revendications précédentes, dans lequel la section distale (7) de l'élément d'endoscopie à fluorescence (27) comporte, côté distal, une LED permettant de découpler la lumière d'excitation et/ou côté distal, un capteur d'image ou une photodiode permettant de coupler la lumière fluorescente.
